(19) Europäisches Patentamt
European Patent Office
Office européen des brevets

(11) Publication number: **0 627 423 A1**

(12) # EUROPEAN PATENT APPLICATION published in accordance with Art. 158(3) EPC

(21) Application number: **93902506.0**

(22) Date of filing: **20.01.93**

(86) International application number:
**PCT/JP93/00065**

(87) International publication number:
**WO 93/15060 (05.08.93 93/19)**

(51) Int. Cl.5: **C07D 231/18**, C07D 231/20, C07D 231/44, C07D 231/46, C07D 401/06, C07D 401/10, C07D 401/12, C07D 403/06, C07D 403/10, C07D 403/12, C07D 413/06

(30) Priority: **22.01.92 JP 9208/92**
**16.12.92 JP 336260/92**

(43) Date of publication of application:
**07.12.94 Bulletin 94/49**

(84) Designated Contracting States:
**AT BE CH DE DK ES FR GB GR IE IT LI LU MC NL PT SE**

(71) Applicant: **NISSAN CHEMICAL INDUSTRIES, LIMITED**
**7-1, Kanda-Nishiki-cho 3-chome**
**Chiyoda-ku Tokyo 101 (JP)**

(72) Inventor: **NAKAJIMA, Yasuyuki, Nissan Chemical Industries Ltd**
**Central Research Institute.,**
**722-1, Tsuboi-cho**
**Funabashi-shi, Chiba-ken 274 (JP)**
Inventor: **WATANABE, Junichi, Nissan Chemical Industries, Ltd**
**Central Research Institute,**
**722-1, Tsuboi-cho**
**Funabashi-shi, Chiba-ken 274 (JP)**
Inventor: **HIROHARA, Yohji, Nissan Chemical Industries, Ltd.**
**Central Research Institute,**
**722-1, Tsuboi-cho**
**Funabashi-shi, Chiba-ken 274 (JP)**
Inventor: **SUZUKI, Hideo, Nissan Chemical Industries, Ltd.**
**Central Research Institute,**
**722-1, Tsuboi-cho**
**Funabashi-shi, Chiba-ken 274 (JP)**
Inventor: **SUGIYAMA, Yasuhisa, Nissan Chemical Industries Ltd**
**Central Research Institute,**
**722-1, Tsuboi-cho**
**Funabashi-shi, Chiba-ken 274 (JP)**
Inventor: **FURUSATO, T., Nissan Chem. Ind. Ltd Seibutsukagaku**
**Kenkyujo,**
**1470, Ohaza Shiraoka,**
**Shiraoka-cho**
**Minamisaitama-gun Saitama-ken 349-02 (JP)**
Inventor: **OYA, H., Nissan Chem Ind Ltd**
**Seibutsukagaku**
**Kenkyujo,**
**1470, Ohaza Shiraoka,**
**Shiraoka-cho**
**Minamisaitama-gun, Saitama-ken 349-02 (JP)**
Inventor: **SASABE, S., Nissan Chem. Ind. Ltd,**
**Seibutsukagaku**
**Kenkyujo,**
**1470, Ohaza Shiraoka,**
**Shiraoka-cho**
**Minamisaitama-gun, Saitama-ken 349-02 (JP)**
Inventor: **NAKAYAMA, M., Nissan Chemical Ind. Seibutsukagaku**
**Kenkyujo,**
**1470, Ohaza Shiraoka,**
**Shiraoka-cho**
**Minamisaitama-gun, Saitama-ken 349-02 (JP)**
Inventor: **HANAUE, M., Nissan Chem. Ind. Ltd.**
**Seibutsukagaku**
**Kenkyujo,**
**1470, Ohaza Shiraoka,**
**Shiraoka-cho**
**Minamisaitama-gun, Saitama-ken 349-02 (JP)**

(74) Representative: **Hansen, Bernd, Dr. Dipl.-Chem. et al**

EP 0 627 423 A1

**Hoffmann, Eitle & Partner,**
**Patentanwälte,**
**Arabellastrasse 4**
**D-81925 München (DE)**

(54) **SUBSTITUTED PYRAZOLE DERIVATIVE AND AGROHORTICULTURAL BACTERICIDE.**

(57) A novel substituted pyrazole derivative represented by general formula (I), a process for the production thereof, and an agrohorticultural bactericide containing the same and not injuring useful crop. In the said formula, $R^1$ represents hydrogen, halogen, alkyl, alkoxy, alkylthio, haloalkyl, cyano, alkoxycarbonyl or optionally substituted phenyl; $R^2$ represents hydrogen, halogen, alkyl, alkoxy, optionally substituted phenyl or phenylalkyl, acyl, ester or amido; A represents optionally substituted phenyl; B represents optionally substituted heterocyclic; X and Y represent each -O-, $-S(O)_{o-2}-$ or $-NR^3-$ wherein $R^3$ represents hydrogen, optionally substituted aliphatic or aromatic group or acyl, or alternatively X represents -CO- or optionally substituted alkylene.

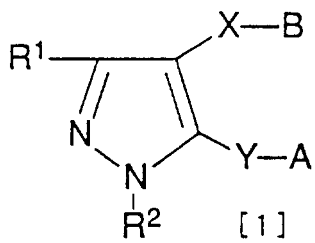

[1]

## TECHNICAL FIELD

The present application relates to novel pyrazole derivatives and phytopathogenic microbicides containing the derivatives as the active ingredients.

## BACKGROUND ART

Various microbicides have heretofore been developed but it is impossible to say that they are always satisfactory in view of their potency and microbes resistant to them.

JP-A 1-125379, EP-459333A1, JP-A 3-141276 and WO-9208715A1 have described that pyrazole derivatives of some kinds have microbicidal activity. (The term "JP-A" as used herein means an "unexamined published Japanese patent application".)

However, the compounds described in the above-mentioned laid-open patent publications are not still satisfactory in view of their potency, their aftereffect, the damage to crops from them, etc. Therefore, the development of phytopathogenic microbicides which are more effective against plant diseases is desired.

## DISCLOSURE OF THE INVENTION

We, the present inventors, considering these situations, assiduously investigated so as to develop compounds having excellent microbicidal activity and, as a result, have found that substituted pyrazole derivatives represented by the following general formula [1] have excellent microbicidal activity and have completed the present invention.

Specifically, the present invention relates to substituted pyrazole derivatives of a general formula [1]:

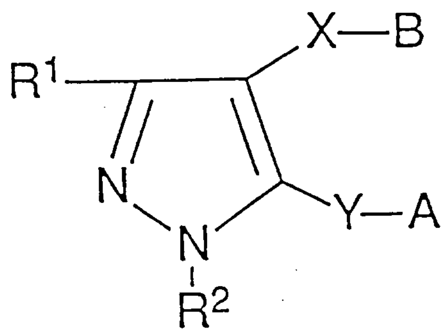

[1]

wherein $R^1$ represents a hydrogen atom, a halogen atom, an alkyl group, an alkoxy group, an alkylthio group, a haloalkyl group, a cyano group, an alkoxycarbonyl group, or an unsubstituted or substituted phenyl group;

$R^2$ represents a hydrogen atom, an alkyl group, a haloalkyl group, an unsubstituted or substituted phenyl group, an unsubstituted or substituted phenylalkyl group, $-COR^6$, or $-SO_2R^7$;

X represents an oxygen atom, -S-, -SO-, $-SO_2-$, $-N(R^3)-$, -CO-, or $-C(R^4)(R^5)-$;

Y represents an oxygen atom, -S-, -SO-, $-SO_2-$, or $-N(R^3)-$;

$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, an unsubstituted or substituted phenylalkyl group, $-COR^6$, or $-SO_2R^7$;

$R^4$ and $R^5$ each independently represent a hydrogen atom, a halogen atom, an alkyl group, a haloalkyl group, an alkenyl group, an alkynyl group, or $-OR^8$;

$R^8$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, an unsubstituted or substituted phenylalkyl group, $-COR^6$, or $-SO_2R^7$;

$R^6$ represents a hydrogen atom, an alkyl group, a haloalkyl group, an unsubstituted or substituted phenyl group, an unsubstituted or substituted phenylalkyl group, an alkoxy group, or $-N(R^9)(R^{10})$;

$R^7$ represents an alkyl group, a haloalkyl group, an unsubstituted or substituted phenyl group, or $-N(R^9)-(R^{10})$;

$R^9$ and $R^{10}$ each independently represent a hydrogen atom, an alkyl group, or an unsubstituted or substituted phenyl group;

A represents an unsubstituted or substituted phenyl group; and
B represents an unsubstituted or substituted heterocyclic group,
and their salts, and also to methods for producing them and phytopathogenic microbicides containing them as the active ingredients.

Of pyrazole derivatives of formula [1], preferred are those where X is an oxygen atom or -N($R^3$)- and their salts.

Of these, more preferred are those of formula [1] where X is an oxygen atom or -N($R^3$)- and Y is -S- or an oxygen atom and their salts.

Further preferred are those of formula [1] where $R^1$ is a lower alkyl group or a halogen atom, $R^2$ is a lower alkyl group, X is an oxygen atom or -N($R^3$)-, Y is -S-, and B is an unsubstituted or substituted pyridyl group or an unsubstituted or substituted pyrimidyl group, and their salts.

The substituents in the above-mentioned general formula, referred to herein, have the following means:

The halogen atom includes fluorine, chlorine, bromine and iodine atoms. Preferred are fluorine, chlorine and bromine atoms.

The alkyl moiety in the alkyl, alkoxy and alkylthio groups has from 1 to 6 carbon atoms. These groups include, for example, methyl, ethyl, n- or i-propyl, n-, s-, i- or t-butyl, pentyl, hexyl, methoxy, ethoxy, n- or i-propoxy, n-, s-, i- or t-butoxy, pentoxy, hexoxy, methylthio, ethylthio, n- or i-propylthio, and n-, s-, i- or t-butylthio groups. Preferred are methyl, ethyl, n- or i-propyl, n-, se-, i- or t-butyl, methoxy, ethoxy, n- or i-propoxy, and n-, s-, i- or t-butoxy groups.

The alkyl moiety in the haloalkyl group has from 1 to 6 carbon atoms, and the group includes, for example, fluoromethyl, difluoromethyl, trifluoromethyl, chloromethyl, bromomethyl, trifluoromethyl, trichloroethyl and trifluoropropyl groups.

The substituted phenyl group has from 1 to 5 same or different substituents. As examples of the substituents, mentioned are a halogen atom, an alkyl group, a haloalkyl group, an alkoxy group, an alkylthio group, a nitro group, a cyano group. Therefore mentioned is a phenyl group substituted by one or more substituents chosen from among them.

The heterocyclic group means a 4-membered to 8-membered ring having one or more hetero atoms such as nitrogen, oxygen and sulfur atoms in addition to carbon atoms. It is preferably a 5-membered or 6-membered ring. More preferably, it is a nitrogen-containing, 5-membered or 6-membered ring. As examples, mentioned are thiazole, oxazole, pyrazole, imidazole, triazole, pyridine, pyrimidine, pyrazine, pyridazine, triazine and tetrazine rings. In the above-mentioned definitions, n means normal, i means iso, s means secondary and t means tertiary.

Specific examples of compounds of formula [1] of the present invention are shown in Table 1 below, which, however, are not limitative. In the following table, Ph means a phenyl group, i means iso, and t means tertiary.

Table 1

In compounds of:

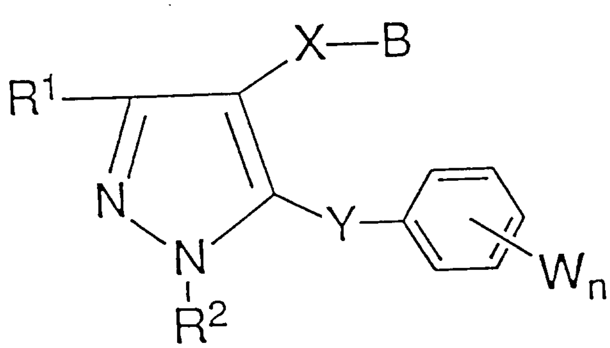

| $R^1$ | $R^2$ | X | Y | $W_n$ | B |
|---|---|---|---|---|---|
| $CH_3$ | $CH_3$ | NH | S | H | B 1 |
| $CF_3$ | $CH_3$ | NH | S | H | B 1 |
| H | $CH_3$ | NH | S | H | B 1 |
| $CH_3$ | $CH_3$ | NH | S | 4-Cl | B 1 |
| $CH_3$ | $CH_3$ | NH | S | 4-$CH_3$ | B 1 |
| $CF_3$ | $CH_3$ | NH | S | 4-$CH_3$ | B 1 |
| H | $CH_3$ | NH | S | 4-$CH_3$ | B 1 |
| $CH_3$ | $CH_3$ | NH | S | 4-F | B 1 |
| $CH_3$ | $CH_3$ | NH | S | 4-Br | B 1 |
| $CH_3$ | $CH_3$ | NH | S | 4-I | B 1 |
| $CH_3$ | $CH_3$ | NH | S | 2-F | B 1 |
| $CH_3$ | $CH_3$ | NH | S | 2-Cl | B 1 |
| $CH_3$ | $CH_3$ | NH | S | 2-Br | B 1 |
| $CH_3$ | $CH_3$ | NH | S | 3-F | B 1 |
| $CH_3$ | $CH_3$ | NH | S | 3-Cl | B 1 |
| $CH_3$ | $CH_3$ | NH | S | 3-Br | B 1 |
| $CH_3$ | $CH_3$ | NH | S | 3-I | B 1 |
| $CH_3$ | $CH_3$ | NH | S | 2-$OCH_3$ | B 1 |
| $CH_3$ | $CH_3$ | NH | S | 3-$OCH_3$ | B 1 |

Table 1 (continued)

| $R^1$ | $R^2$ | X | Y | $W_n$ | B |
|---|---|---|---|---|---|
| $CH_3$ | $CH_3$ | NH | S | 4-$OCH_3$ | B 1 |
| $CH_3$ | $CH_3$ | NH | S | 2-$CF_3$ | B 1 |
| $CH_3$ | $CH_3$ | NH | S | 3-$CF_3$ | B 1 |
| $CH_3$ | $CH_3$ | NH | S | 4-$CF_3$ | B 1 |
| $CH_3$ | $CH_3$ | NH | S | 4-$C_2H_5$ | B 1 |
| $CH_3$ | $CH_3$ | NH | S | 4-$C_3H_7$ | B 1 |
| $CH_3$ | $CH_3$ | NH | S | 4-$C_4H_9$ | B 1 |
| $CH_3$ | $CH_3$ | NH | S | 4-i-$C_3H_7$ | B 1 |
| $CH_3$ | $CH_3$ | NH | S | 4-t-$C_4H_9$ | B 1 |
| $CH_3$ | $CH_3$ | NH | S | 2-$OCF_3$ | B 1 |
| $CH_3$ | $CH_3$ | NH | S | 3-$OCF_3$ | B 1 |
| $CH_3$ | $CH_3$ | NH | S | 4-$OCF_3$ | B 1 |
| $CH_3$ | $CH_3$ | NH | S | 3-$NO_2$ | B 1 |
| $CH_3$ | $CH_3$ | NH | S | 4-$NO_2$ | B 1 |
| $CH_3$ | $CH_3$ | NH | S | 3-$NH_2$ | B 1 |
| $CH_3$ | $CH_3$ | NH | S | 4-$NH_2$ | B 1 |
| $CH_3$ | $CH_3$ | NH | S | 4-$NHCOCH_3$ | B 1 |
| $CH_3$ | $CH_3$ | NH | S | 4-$NHSO_2CH_3$ | B 1 |

Table 1 (continued)

| $R^1$ | $R^2$ | X | Y | $W_n$ | B |
|---|---|---|---|---|---|
| $CH_3$ | $CH_3$ | NH | S | 4-$NHCOCF_3$ | B 1 |
| $CH_3$ | $CH_3$ | NH | S | 4-$NHSO_2CF_3$ | B 1 |
| $CH_3$ | $CH_3$ | NH | S | 4-Ph | B 1 |
| $CH_3$ | $CH_3$ | NH | S | 4-OPh | B 1 |
| $CH_3$ | $CH_3$ | NH | S | 4-$OCF_2CF_2H$ | B 1 |
| $CH_3$ | $CH_3$ | NH | S | 4-$COCH_3$ | B 1 |
| $CH_3$ | $CH_3$ | NH | S | 4-NHCOPh | B 1 |
| $CH_3$ | $CH_3$ | NH | S | 4-$NHCOOCH_3$ | B 1 |
| $CH_3$ | $CH_3$ | NH | S | 4-$NHCON(CH_3)_2$ | B 1 |
| $CH_3$ | $CH_3$ | NH | S | 2-Cl, 3-Cl | B 1 |
| $CH_3$ | $CH_3$ | NH | S | 2-Cl, 4-Cl | B 1 |
| $CH_3$ | $CH_3$ | NH | S | 2-Cl, 5-Cl | B 1 |
| $CH_3$ | $CH_3$ | NH | S | 2-Cl, 6-Cl | B 1 |
| $CH_3$ | $CH_3$ | NH | S | 3-Cl, 4-Cl | B 1 |
| $CH_3$ | $CH_3$ | NH | S | 3-Cl, 5-Cl | B 1 |
| $CH_3$ | $CH_3$ | NH | S | 2-$CH_3$, 3-$CH_3$ | B 1 |
| $CH_3$ | $CH_3$ | NH | S | 2-$CH_3$, 4-$CH_3$ | B 1 |
| $CH_3$ | $CH_3$ | NH | S | 2-$CH_3$, 5-$CH_3$ | B 1 |

Table 1 (continued)

| $R^1$ | $R^2$ | X | Y | $W_n$ | B |
|---|---|---|---|---|---|
| $CH_3$ | $CH_3$ | NH | S | 2-$CH_3$, 6-$CH_3$ | B 1 |
| $CH_3$ | $CH_3$ | NH | S | 3-$CH_3$, 4-$CH_3$ | B 1 |
| $CH_3$ | $CH_3$ | NH | S | 3-$CH_3$, 5-$CH_3$ | B 1 |
| $CH_3$ | $CH_3$ | NH | S | 2-Cl, 3-$CH_3$ | B 1 |
| $CH_3$ | $CH_3$ | NH | S | 2-Cl, 4-$CH_3$ | B 1 |
| $CH_3$ | $CH_3$ | NH | S | 2-Cl, 5-$CH_3$ | B 1 |
| $CH_3$ | $CH_3$ | NH | S | 3-Cl, 4-$CH_3$ | B 1 |
| $CH_3$ | $CH_3$ | NH | S | 3-Cl, 5-$CH_3$ | B 1 |
| $CH_3$ | $CH_3$ | NH | S | 2-$CH_3$, 3-Cl | B 1 |
| $CH_3$ | $CH_3$ | NH | S | 2-$CH_3$, 4-Cl | B 1 |
| $CH_3$ | $CH_3$ | NH | S | 2-$CH_3$, 5-Cl | B 1 |
| $CH_3$ | $CH_3$ | NH | S | 2-F, 3-F | B 1 |
| $CH_3$ | $CH_3$ | NH | S | 2-F, 4-F | B 1 |
| $CH_3$ | $CH_3$ | NH | S | 2-F, 5-F | B 1 |
| $CH_3$ | $CH_3$ | NH | S | 2-F, 6-F | B 1 |
| $CH_3$ | $CH_3$ | NH | S | 2-F, 3-Cl | B 1 |
| $CH_3$ | $CH_3$ | NH | S | 2-F, 4-Cl | B 1 |
| $CH_3$ | $CH_3$ | NH | S | 2-F, 5-Cl | B 1 |

Table 1 (continued)

| $R^1$ | $R^2$ | X | Y | $W_n$ | B |
|---|---|---|---|---|---|
| $CH_3$ | $CH_3$ | NH | S | 2-F, 6-Cl | B 1 |
| $CH_3$ | $CH_3$ | NH | S | 2-F, 3-$CH_3$ | B 1 |
| $CH_3$ | $CH_3$ | NH | S | 2-F, 4-$CH_3$ | B 1 |
| $CH_3$ | $CH_3$ | NH | S | 2-F, 3-Br | B 1 |
| $CH_3$ | $CH_3$ | NH | S | 2-F, 4-Br | B 1 |
| $CH_3$ | $CH_3$ | NH | S | 3-F, 4-Cl | B 1 |
| $CH_3$ | $CH_3$ | NH | S | 3-Cl, 4-F | B 1 |
| $CH_3$ | $CH_3$ | NH | S | 3-F, 4-$CH_3$ | B 1 |
| $CH_3$ | $CH_3$ | NH | S | 3-F, 5-$CH_3$ | B 1 |
| $CH_3$ | $CH_3$ | NH | S | 2-Br, 4-Cl | B 1 |
| $CH_3$ | $CH_3$ | NH | S | 2-Br, 4-Br | B 1 |
| $CH_3$ | $CH_3$ | NH | S | 2-Br, 4-$CH_3$ | B 1 |
| $CH_3$ | $CH_3$ | NH | S | 3-Br, 4-Cl | B 1 |
| $CH_3$ | $CH_3$ | NH | S | 3-Br, 4-Br | B 1 |
| $CH_3$ | $CH_3$ | NH | S | 3-Br, 4-$CH_3$ | B 1 |
| $CH_3$ | $CH_3$ | NH | S | 2-Cl, 4-Br | B 1 |
| $CH_3$ | $CH_3$ | NH | S | 3-Cl, 4-Br | B 1 |
| $CH_3$ | $CH_3$ | NH | S | 3-$CH_3$, 4-Br | B 1 |

Table 1 (continued)

| $R^1$ | $R^2$ | X | Y | $W_n$ | B |
|---|---|---|---|---|---|
| $CH_3$ | $CH_3$ | NH | S | 3-Cl, 4-I | B 1 |
| $CH_3$ | $CH_3$ | NH | S | 3-F, 4-Br | B 1 |
| $CH_3$ | $CH_3$ | NH | S | 3, 4-$OCH_2O$- | B 1 |
| $CH_3$ | $CH_3$ | NH | S | 2-$CF_3$, 3-Cl | B 1 |
| $CH_3$ | $CH_3$ | NH | S | 2-$CF_3$, 4-Cl | B 1 |
| $CH_3$ | $CH_3$ | NH | S | 2-$CF_3$, 3-Br | B 1 |
| $CH_3$ | $CH_3$ | NH | S | 2-$CF_3$, 4-Br | B 1 |
| $CH_3$ | $CH_3$ | NH | S | 2-F, 4-$NO_2$ | B 1 |
| $CH_3$ | $CH_3$ | NH | S | 3-F, 4-$NO_2$ | B 1 |
| $CH_3$ | $CH_3$ | NH | S | 2-Cl, 4-$NO_2$ | B 1 |
| $CH_3$ | $CH_3$ | NH | S | 3-Cl, 4-$NO_2$ | B 1 |
| $CH_3$ | $CH_3$ | NH | S | 2-F, 4-$OCH_3$ | B 1 |
| $CH_3$ | $CH_3$ | NH | S | 3-F, 4-$OCH_3$ | B 1 |
| $CH_3$ | $CH_3$ | NH | S | 2-Cl, 4-$OCH_3$ | B 1 |
| $CH_3$ | $CH_3$ | NH | S | 3-Cl, 4-$OCH_3$ | B 1 |
| $CH_3$ | $CH_3$ | NH | S | 2-$OCH_3$, 4-$OCH_3$ | B 1 |
| $CH_3$ | $CH_3$ | NH | S | 3-$OCH_3$, 4-$OCH_3$ | B 1 |
| $CH_3$ | $CH_3$ | NH | S | 2-Cl, 3-Cl, 4-Cl | B 1 |
| $CH_3$ | $CH_3$ | NH | S | 2-Cl, 4-Cl, 5-Cl | B 1 |
| $CH_3$ | $CH_3$ | NH | S | 2-Cl, 4-Cl, 6-Cl | B 1 |

Table 1 (continued)

| $R^1$ | $R^2$ | X | Y | $W_n$ | B |
|---|---|---|---|---|---|
| $CH_3$ | $CH_3$ | NH | S | 3-Cl, 4-Cl, 5-Cl | B 1 |
| $CH_3$ | $CH_3$ | NH | S | 2-Cl, 3-Cl, 4-$CH_3$ | B 1 |
| $CH_3$ | $CH_3$ | NH | S | 2-Cl, 3-Cl, 4-Br | B 1 |
| $CH_3$ | $CH_3$ | NH | S | 2-Cl, 3-Cl, 4-I | B 1 |
| $CH_3$ | $CH_3$ | NH | S | 2-Cl, 4-Br, 6-Cl | B 1 |
| $CH_3$ | $CH_3$ | NH | S | 2-Cl, 4-$CH_3$, 6-Cl | B 1 |
| $CH_3$ | $CH_3$ | NH | S | 2-Br, 4-Cl, 6-Cl | B 1 |
| $CH_3$ | $CH_3$ | NCHO | S | 4-Cl | B 1 |
| $CH_3$ | $CH_3$ | NCHO | S | 2-Cl, 4-Cl | B 1 |
| $CH_3$ | $CH_3$ | $NCOCH_3$ | S | 4-Cl | B 1 |
| $CH_3$ | $CH_3$ | $NCOCH_3$ | S | 2-Cl, 4-Cl | B 1 |
| $CH_3$ | $CH_3$ | $NCOOCH_3$ | S | 2-Cl, 4-Cl | B 1 |
| $CH_3$ | $CH_3$ | $NCON(CH_3)_2$ | S | 2-Cl, 4-Cl | B 1 |
| $CH_3$ | $CH_3$ | $NCH_3$ | S | 4-Cl | B 1 |
| $CH_3$ | $CH_3$ | $NCH_3$ | S | 2-Cl, 4-Cl | B 1 |
| $CH_3$ | $CH_3$ | $NCH_2OCH_3$ | S | 2-Cl, 4-Cl | B 1 |
| $CH_3$ | $CH_3$ | $NCH_2CH=CH_2$ | S | 2-Cl, 4-Cl | B 1 |
| $CH_3$ | $CH_3$ | $NCH_2C\equiv CH$ | S | 2-Cl, 4-Cl | B 1 |
| $CH_3$ | $CH_3$ | $NCH_2COCH_3$ | S | 2-Cl, 4-Cl | B 1 |
| $CH_3$ | $CH_3$ | $NCH_2COOCH_3$ | S | 2-Cl, 4-Cl | B 1 |

Table 1 (continued)

| $R^1$ | $R^2$ | X | Y | $W_n$ | B |
|---|---|---|---|---|---|
| $CH_3$ | $CH_3$ | $NCH_2CN$ | S | 2-Cl, 4-Cl | B 1 |
| $CH_3$ | $CH_3$ | $NCH_2Ph$ | S | 4-Cl | B 1 |
| $CH_3$ | $CH_3$ | $NCH_2Ph$ | S | 2-Cl, 4-Cl | B 1 |
| $CH_3$ | $CH_3$ | $NCH_2C_6H_4$-4-Cl | S | 2-Cl, 4-Cl | B 1 |
| $CH_3$ | $CH_3$ | $NSO_2CH_3$ | S | 2-Cl, 4-Cl | B 1 |
| $CH_3$ | $CH_3$ | $NSO_2N(CH_3)_2$ | S | 2-Cl, 4-Cl | B 1 |
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | $NCH_3$ | S | 2-Cl, 4-Cl | B 2 |
| $CH_3$ | $CH_3$ | $NCOCH_3$ | S | 2-Cl, 4-Cl | B 2 |
| $CH_3$ | $CH_3$ | $NCH_2Ph$ | S | 2-Cl, 4-Cl | B 2 |
| $CH_3$ | H | NH | S | 4-Cl | B 1 |
| $CH_3$ | H | NH | S | 2-Cl, 4-Cl | B 1 |
| $CH_3$ | H | NH | S | 3-Cl, 4-$CH_3$ | B 1 |
| $CH_3$ | $C_2H_5$ | NH | S | 4-Cl | B 1 |
| $CH_3$ | $C_2H_5$ | NH | S | 4-Br | B 1 |
| $CH_3$ | $C_2H_5$ | NH | S | 2-Cl, 4-Cl | B 1 |
| $CH_3$ | $C_2H_5$ | NH | S | 2-Cl, 4-$CH_3$ | B 1 |
| $CH_3$ | i-$C_3H_7$ | NH | S | 4-Cl | B 1 |
| $CH_3$ | i-$C_3H_7$ | NH | S | 2-Cl, 4-Cl | B 1 |

Table 1 (continued)

| $R^1$ | $R^2$ | X | Y | $W_n$ | B |
|---|---|---|---|---|---|
| $CH_3$ | $t-C_4H_9$ | NH | S | 4-Cl | B 1 |
| $CH_3$ | $t-C_4H_9$ | NH | S | 2-Cl, 4-Cl | B 1 |
| $CH_3$ | $C_6H_5$ | NH | S | 4-Cl | B 1 |
| $CH_3$ | $C_6H_5$ | NH | S | 2-Cl, 4-Cl | B 1 |
| $CH_3$ | $C_6H_5-4-Cl$ | NH | S | 2-Cl, 4-Cl | B 1 |
| $CH_3$ | $CF_3$ | NH | S | 4-Cl | B 1 |
| $CH_3$ | $CF_3$ | NH | S | 4-Br | B 1 |
| $CH_3$ | $CF_3$ | NH | S | 2-Cl, 4-Cl | B 1 |
| $CH_3$ | $CF_3$ | NH | S | 3-Cl, 4-$CH_3$ | B 1 |
| $CH_3$ | H | NH | S | 4-Cl | B 2 |
| $CH_3$ | H | NH | S | 2-Cl, 4-Cl | B 2 |
| $CH_3$ | $C_2H_5$ | NH | S | 4-Cl | B 2 |
| $CH_3$ | $i-C_3H_7$ | NH | S | 4-Cl | B 2 |
| $CH_3$ | $t-C_4H_9$ | NH | S | 4-Cl | B 2 |
| $CH_3$ | $CF_3$ | NH | S | 2-Cl, 4-Cl | B 2 |
| $CH_3$ | $CH_2Ph$ | NH | S | 4-Cl | B 1 |
| $CH_3$ | $CH_2Ph$ | NH | S | 2-Cl, 4-Cl | B 1 |
| $CH_3$ | $CH_2Ph$ | NCHO | S | 2-Cl, 4-Cl | B 1 |
| $CH_3$ | CHO | NH | S | 4-Cl | B 1 |
| $CH_3$ | CHO | NCHO | S | 4-Cl | B 1 |

Table 1 (continued)

| $R^1$ | $R^2$ | X | Y | $W_n$ | B |
|---|---|---|---|---|---|
| $CH_3$ | CHO | NH | S | 2-Cl, 4-Cl | B 1 |
| $CH_3$ | CHO | NCHO | S | 2-Cl, 4-Cl | B 1 |
| $CH_3$ | $COCH_3$ | NH | S | 4-Cl | B 1 |
| $CH_3$ | $COCH_3$ | NH | S | 4-Br | B 1 |
| $CH_3$ | $COCH_3$ | NH | S | 2-Cl, 4-Cl | B 1 |
| $CH_3$ | $COCH_3$ | NCHO | S | 2-Cl, 4-Cl | B 1 |
| $CH_3$ | $SO_2CH_3$ | NH | S | 4-Cl | B 1 |
| $CH_3$ | $SO_2CH_3$ | NH | S | 2-Cl, 4-Cl | B 1 |
| $CH_3$ | $SO_2CH_3$ | NH | S | 3-Cl, 4-$CH_3$ | B 1 |
| $CH_3$ | $CONHCH_3$ | NH | S | 4-Cl | B 1 |
| $CH_3$ | $CONHCH_3$ | NH | S | 4-Br | B 1 |
| $CH_3$ | $CONHCH_3$ | NH | S | 2-Cl, 4-Cl | B 1 |
| $CH_3$ | $CONHCH_3$ | NH | S | 3-Cl, 4-$CH_3$ | B 1 |
| $CH_3$ | $CH_2Ph$ | NH | S | 4-Cl | B 2 |
| $CH_3$ | $CH_2Ph$ | NH | S | 2-Cl, 4-Cl | B 2 |
| $CH_3$ | $COCH_3$ | NH | S | 4-Cl | B 2 |
| $CH_3$ | $CONHCH_3$ | NH | S | 4-Cl | B 2 |
| $CH_3$ | $SO_2CH_3$ | NH | S | 4-Cl | B 2 |
| $CH_3$ | $SO_2CH_3$ | NH | S | 2-Cl, 4-Cl | B 2 |
| H | $CH_3$ | NH | S | 4-Cl | B 1 |

Table 1 (continued)

| $R^1$ | $R^2$ | X | Y | $W_n$ | B |
|---|---|---|---|---|---|
| H | $CH_3$ | NH | S | 2-Cl, 4-Cl | B 1 |
| H | $CH_3$ | NH | S | 3-Cl, 4-$CH_3$ | B 1 |
| $CF_3$ | $CH_3$ | NH | S | 4-Cl | B 1 |
| $CF_3$ | $CH_3$ | NH | S | 3-Cl, 4-$CH_3$ | B 1 |
| $CF_3$ | $CH_3$ | NH | S | 2-Cl, 4-Cl | B 1 |
| $CF_3$ | $CH_3$ | NH | S | 2-Cl, 4-$CH_3$ | B 1 |
| $C_2H_5$ | $CH_3$ | NH | S | 4-Cl | B 1 |
| $C_2H_5$ | $CH_3$ | NH | S | 2-Cl, 4-Cl | B 1 |
| $C_2H_5$ | $CH_3$ | NH | S | 2-Cl, 4-$CH_3$ | B 1 |
| i-$C_3H_7$ | $CH_3$ | NH | S | 4-Cl | B 1 |
| i-$C_3H_7$ | $CH_3$ | NH | S | 2-Cl, 4-Cl | B 1 |
| t-$C_4H_9$ | $CH_3$ | NH | S | 4-Cl | B 1 |
| t-$C_4H_9$ | $CH_3$ | NH | S | 2-Cl, 4-Cl | B 1 |
| $C_6H_5$ | $CH_3$ | NH | S | 4-Cl | B 1 |
| $C_6H_5$ | $CH_3$ | NH | S | 2-Cl, 4-Cl | B 1 |
| 2, 6-$Cl_2$-$C_6H_5$ | $CH_3$ | NH | S | 4-Cl | B 1 |
| 2, 6-$Cl_2$-$C_6H_5$ | $CH_3$ | NH | S | 2-Cl, 4-Cl | B 1 |
| H | $CH_3$ | NH | S | 4-Cl | B 2 |
| H | $CH_3$ | NH | S | 2-Cl, 4-Cl | B 2 |
| $C_2H_5$ | $CH_3$ | NH | S | 2-Cl, 4-Cl | B 2 |

Table 1 (continued)

| $R^1$ | $R^2$ | X | Y | $W_n$ | B |
|---|---|---|---|---|---|
| i-$C_3H_7$ | $CH_3$ | NH | S | 2-Cl, 4-Cl | B 2 |
| $CF_3$ | $CH_3$ | NH | S | 4-Cl | B 2 |
| $CF_3$ | $CH_3$ | NH | S | 2-Cl, 4-Cl | B 2 |
| F | $CH_3$ | NH | S | 2-Cl, 4-Cl | B 1 |
| Cl | $CH_3$ | NH | S | 4-Cl | B 1 |
| Cl | $CH_3$ | NH | S | 4-$CH_3$ | B 1 |
| Cl | $CH_3$ | NH | S | 2-Cl, 4-Cl | B 1 |
| Cl | $CH_3$ | NH | S | 3-Cl, 4-Cl | B 1 |
| Cl | $CH_3$ | NH | S | 2-F, 4-Cl | B 1 |
| Cl | $CH_3$ | NH | S | 3-F, 4-Cl | B 1 |
| Cl | $CH_3$ | NH | S | 2-F, 4-$CH_3$ | B 1 |
| Cl | $CH_3$ | NH | S | 3-F, 4-$CH_3$ | B 1 |
| Cl | $CH_3$ | NH | S | 2-Cl, 4-$CH_3$ | B 1 |
| Cl | $CH_3$ | NH | S | 3-Cl, 4-$CH_3$ | B 1 |
| Cl | $CH_3$ | NH | S | 3-Cl, 4-$OCH_3$ | B 1 |
| Cl | $CH_3$ | NH | S | 3-F, 4-$OCH_3$ | B 1 |
| Br | $CH_3$ | NH | S | 2-Cl, 4-Cl | B 1 |
| Cl | $CH_3$ | NCHO | S | 4-Cl | B 1 |
| Cl | $CH_3$ | NCHO | S | 2-Cl, 4-Cl | B 1 |
| Cl | $CH_3$ | NCHO | S | 2-Cl, 4-$CH_3$ | B 1 |

Table 1 (continued)

| $R^1$ | $R^2$ | X | Y | $W_n$ | B |
|---|---|---|---|---|---|
| Cl | $CH_3$ | NCHO | S | 3-Cl, 4-$CH_3$ | B 1 |
| Cl | $CH_3$ | NCHO | S | 3-F, 4-$CH_3$ | B 1 |
| Cl | $CH_3$ | NCHO | S | 3-Cl, 4-$OCH_3$ | B 1 |
| Cl | $CH_3$ | NH | S | 4-Cl | B 2 |
| Cl | $CH_3$ | NH | S | 2-Cl, 4-Cl | B 2 |
| Cl | $CH_3$ | NH | S | 3-Cl, 4-$CH_3$ | B 2 |
| Cl | $CH_3$ | NCHO | S | 3-Cl, 4-$CH_3$ | B 2 |
| Cl | $CH_3$ | NH | S | 2-Cl, 4-Cl | B 3 |
| Cl | $CH_3$ | NH | S | 2-Cl, 4-Cl | B 4 |
| Cl | $CH_3$ | NH | S | 2-Cl, 4-Cl | B 5 |
| Cl | $CH_3$ | NH | S | 2-Cl, 4-Cl | B 6 |
| Cl | $CH_3$ | NH | S | 2-Cl, 4-Cl | B 7 |
| Cl | $CH_3$ | NH | S | 2-Cl, 4-Cl | B 10 |
| Cl | $CH_3$ | NH | S | 2-Cl, 4-Cl | B 11 |
| Cl | $CH_3$ | NH | S | 2-Cl, 4-Cl | B 13 |
| Cl | $CH_3$ | NH | S | 2-Cl, 4-Cl | B 16 |
| Cl | $CH_3$ | NH | S | 2-Cl, 4-Cl | B 17 |
| Cl | $CH_3$ | NH | S | 2-Cl, 4-Cl | B 18 |
| $CH_3O$ | $CH_3$ | NH | S | 4-Cl | B 1 |
| $CH_3O$ | $CH_3$ | NH | S | 2-Cl, 4-Cl | B 1 |

Table 1 (continued)

| $R^1$ | $R^2$ | X | Y | $W_n$ | B |
|---|---|---|---|---|---|
| $CH_3O$ | $CH_3$ | NH | S | 2-Cl, 4-$CH_3$ | B 1 |
| $CH_3O$ | $CH_3$ | NCHO | S | 4-Cl | B 1 |
| $CH_3O$ | $CH_3$ | NCHO | S | 2-Cl, 4-Cl | B 1 |
| $CH_3O$ | $CH_3$ | NCHO | S | 3-Cl, 4-$CH_3$ | B 1 |
| $CH_3S$ | $CH_3$ | NH | S | 4-Cl | B 1 |
| $CH_3S$ | $CH_3$ | NH | S | 2-Cl, 4-Cl | B 1 |
| $CH_3S$ | $CH_3$ | NH | S | 3-Cl, 4-$CH_3$ | B 1 |
| $CH_3S$ | $CH_3$ | NCHO | S | 4-Cl | B 1 |
| $CH_3S$ | $CH_3$ | NCHO | S | 2-Cl, 4-Cl | B 1 |
| $CH_3O$ | $CH_3$ | NH | S | 2-Cl, 4-Cl | B 2 |
| $CH_3S$ | $CH_3$ | NH | S | 2-Cl, 4-Cl | B 2 |
| $CH_3$ | $CH_3$ | NH | SO | 4-Cl | B 1 |
| $CH_3$ | $CH_3$ | NH | $SO_2$ | 4-Cl | B 1 |
| $CH_3$ | $CH_3$ | NH | SO | 2-Cl, 4-Cl | B 1 |
| $CH_3$ | $CH_3$ | NH | $SO_2$ | 2-Cl, 4-Cl | B 1 |
| $CH_3$ | $CH_3$ | NH | SO | 3-Cl, 4-$CH_3$ | B 1 |
| $CH_3$ | $CH_3$ | NH | $SO_2$ | 3-Cl, 4-$CH_3$ | B 1 |
| $CH_3$ | $CH_3$ | NH | O | 4-Cl | B 1 |
| $CH_3$ | $CH_3$ | NH | O | 4-$CH_3$ | B 1 |
| $CH_3$ | $CH_3$ | NH | O | 4-$C_2H_5$ | B 1 |

Table 1 (continued)

| $R^1$ | $R^2$ | X | Y | $W_n$ | B |
|---|---|---|---|---|---|
| $CH_3$ | $CH_3$ | NH | O | 4-$OCH_3$ | B 1 |
| $CH_3$ | $CH_3$ | NH | O | 2-Cl, 4-Cl | B 1 |
| $CH_3$ | $CH_3$ | NH | O | 3-Cl, 4-Cl | B 1 |
| $CH_3$ | $CH_3$ | NH | O | 2-Cl, 4-$CH_3$ | B 1 |
| $CH_3$ | $CH_3$ | NH | O | 3-Cl, 4-$CH_3$ | B 1 |
| $CH_3$ | $CH_3$ | NH | O | 2-F, 4-Cl | B 1 |
| $CH_3$ | $CH_3$ | NH | O | 3-$CH_3$, 4-$CH_3$ | B 1 |
| $CH_3$ | $CH_3$ | NCHO | O | 4-Cl | B 1 |
| $CH_3$ | $CH_3$ | $NCH_3$ | O | 4-Cl | B 1 |
| $CH_3$ | $CH_3$ | NCHO | O | 2-Cl, 4-Cl | B 1 |
| $CH_3$ | $CH_3$ | $NCH_3$ | O | 2-Cl, 4-Cl | B 1 |
| $CH_3$ | $CH_3$ | NH | O | 4-Cl | B 2 |
| $CH_3$ | $CH_3$ | NH | O | 4-$CH_3$ | B 2 |
| $CH_3$ | $CH_3$ | NH | O | 2-Cl, 4-Cl | B 2 |
| $CH_3$ | $CH_3$ | NH | O | 3-Cl, 4-Cl | B 2 |
| $CH_3$ | $CH_3$ | NH | S | 4-F | B 2 |
| $CH_3$ | $CH_3$ | NH | S | 4-Cl | B 2 |
| $CH_3$ | $CH_3$ | NH | S | 4-$CH_3$ | B 2 |
| $CH_3$ | $CH_3$ | NH | S | 2-Cl, 4-Cl | B 2 |
| $CH_3$ | $CH_3$ | NH | S | 3-Cl, 4-Cl | B 2 |

Table 1 (continued)

| $R^1$ | $R^2$ | X | Y | $W_n$ | B |
|---|---|---|---|---|---|
| $CH_3$ | $CH_3$ | NH | S | 2-Cl, 4-$CH_3$ | B 2 |
| $CH_3$ | $CH_3$ | NH | S | 3-Cl, 4-$CH_3$ | B 2 |
| $CH_3$ | $CH_3$ | NH | S | 2-F, 4-F | B 2 |
| $CH_3$ | $CH_3$ | NH | S | 2-F, 4-Cl | B 2 |
| $CH_3$ | $CH_3$ | NH | S | 3-F, 4-Cl | B 2 |
| $CH_3$ | $CH_3$ | NH | S | 2-F, 4-$CH_3$ | B 2 |
| $CH_3$ | $CH_3$ | NH | S | 3-F, 4-$CH_3$ | B 2 |
| $CH_3$ | $CH_3$ | NCHO | S | 4-Cl | B 2 |
| $CH_3$ | $CH_3$ | NCHO | S | 2-Cl, 4-Cl | B 2 |
| $CH_3$ | $CH_3$ | NCHO | S | 3-Cl, 4-$CH_3$ | B 2 |
| $CH_3$ | $CH_3$ | NH | S | 4-Cl | B 3 |
| $CH_3$ | $CH_3$ | NH | S | 4-Br | B 3 |
| $CH_3$ | $CH_3$ | NH | S | 2-Cl, 4-Cl | B 3 |
| $CH_3$ | $CH_3$ | NH | S | 2-Cl, 4-$CH_3$ | B 3 |
| $CH_3$ | $CH_3$ | NH | S | 3-Cl, 4-$CH_3$ | B 3 |
| $CH_3$ | $CH_3$ | NH | S | 4-Cl | B 4 |
| $CH_3$ | $CH_3$ | NH | S | 2-Cl, 4-Cl | B 4 |
| $CH_3$ | $CH_3$ | NH | S | 4-Cl | B 5 |
| $CH_3$ | $CH_3$ | NH | S | 2-Cl, 4-Cl | B 5 |
| $CH_3$ | $CH_3$ | NH | S | 4-Cl | B 6 |

Table 1 (continued)

| $R^1$ | $R^2$ | X | Y | $W_n$ | B |
|---|---|---|---|---|---|
| $CH_3$ | $CH_3$ | NH | S | 2-Cl, 4-Cl | B 6 |
| $CH_3$ | $CH_3$ | NH | S | 4-Cl | B 7 |
| $CH_3$ | $CH_3$ | NH | S | 2-Cl, 4-$CH_3$ | B 7 |
| $CH_3$ | $CH_3$ | NH | S | 4-Cl | B 8 |
| $CH_3$ | $CH_3$ | NH | S | 2-Cl, 4-Cl | B 8 |
| $CH_3$ | $CH_3$ | NH | S | 4-Cl | B 9 |
| $CH_3$ | $CH_3$ | NH | S | 2-Cl, 4-$CH_3$ | B 9 |
| $CH_3$ | $CH_3$ | NH | S | 4-Cl | B 10 |
| $CH_3$ | $CH_3$ | NH | S | 2-Cl, 4-Cl | B 10 |
| $CH_3$ | $CH_3$ | NH | S | 3-Cl, 4-Cl | B 10 |
| $CH_3$ | $CH_3$ | NH | S | 2-F, 4-$CH_3$ | B 10 |
| $CH_3$ | $CH_3$ | NH | S | 3-F, 4-$CH_3$ | B 10 |
| $CH_3$ | $CH_3$ | NH | S | 2-F, 4-Cl | B 10 |
| $CH_3$ | $CH_3$ | NH | S | 4-Cl | B 11 |
| $CH_3$ | $CH_3$ | NH | S | 2-Cl, 4-$CH_3$ | B 11 |
| $CH_3$ | $CH_3$ | NH | S | 4-Cl | B 12 |
| $CH_3$ | $CH_3$ | NH | S | 2-Cl, 4-Cl | B 12 |
| $CH_3$ | $CH_3$ | NH | S | 2-F, 4-$CH_3$ | B 12 |
| $CH_3$ | $CH_3$ | NH | S | 3-F, 4-$CH_3$ | B 12 |
| $CH_3$ | $CH_3$ | NH | S | 2-Cl, 4-$CH_3$ | B 12 |

Table 1 (continued)

| $R^1$ | $R^2$ | X | Y | $W_n$ | B |
|---|---|---|---|---|---|
| $CH_3$ | $CH_3$ | NH | S | 3-Cl, 4-$CH_3$ | B 1 2 |
| $CH_3$ | $CH_3$ | NCHO | S | 4-Cl | B 1 2 |
| $CH_3$ | $CH_3$ | NCHO | S | 2-Cl, 4-Cl | B 1 2 |
| $CH_3$ | $CH_3$ | NH | S | 4-Cl | B 1 3 |
| $CH_3$ | $CH_3$ | NH | S | 2-Cl, 4-Cl | B 1 3 |
| $CH_3$ | $CH_3$ | NH | S | 2-Cl, 4-$CH_3$ | B 1 3 |
| $CH_3$ | $CH_3$ | NH | S | 2-F, 4-$CH_3$ | B 1 3 |
| $CH_3$ | $CH_3$ | NH | S | 4-Cl | B 1 4 |
| $CH_3$ | $CH_3$ | NH | S | 2-Cl, 4-Cl | B 1 4 |
| $CH_3$ | $CH_3$ | NH | S | 2-Cl, 4-$CH_3$ | B 1 4 |
| $CH_3$ | $CH_3$ | NH | S | 2-F, 4-$CH_3$ | B 1 4 |
| $CH_3$ | $CH_3$ | NH | S | 4-Cl | B 1 5 |
| $CH_3$ | $CH_3$ | NH | S | 2-Cl, 4-Cl | B 1 5 |
| $CH_3$ | $CH_3$ | NH | S | 2-Cl, 4-$CH_3$ | B 1 5 |
| $CH_3$ | $CH_3$ | NH | S | 2-F, 4-$CH_3$ | B 1 5 |
| $CH_3$ | $CH_3$ | NH | S | 4-Cl | B 1 6 |
| $CH_3$ | $CH_3$ | NH | S | 2-Cl, 4-Cl | B 1 6 |
| $CH_3$ | $CH_3$ | NH | S | 2-Cl, 4-$CH_3$ | B 1 6 |
| $CH_3$ | $CH_3$ | NH | S | 2-F, 4-$CH_3$ | B 1 6 |
| $CH_3$ | $CH_3$ | NH | S | 4-Cl | B 1 7 |

Table 1 (continued)

| $R^1$ | $R^2$ | X | Y | $W_n$ | B |
|---|---|---|---|---|---|
| $CH_3$ | $CH_3$ | NH | S | 2-Cl, 4-Cl | B17 |
| $CH_3$ | $CH_3$ | NH | S | 2-Cl, 4-$CH_3$ | B17 |
| $CH_3$ | $CH_3$ | NH | S | 2-F, 4-$CH_3$ | B17 |
| $CH_3$ | $CH_3$ | NH | S | 4-Cl | B18 |
| $CH_3$ | $CH_3$ | NH | S | 2-Cl, 4-Cl | B18 |
| $CH_3$ | $CH_3$ | NH | S | 2-Cl, 4-$CH_3$ | B18 |
| $CH_3$ | $CH_3$ | NH | S | 2-F, 4-$CH_3$ | B18 |
| $CH_3$ | $CH_3$ | NCHO | S | 4-Br | B1 |
| $CH_3$ | $CH_3$ | NCHO | S | 3-Cl, 4-Cl | B1 |
| $CH_3$ | $CH_3$ | NCHO | S | 2-Cl, 5-Cl | B1 |
| $CH_3$ | $CH_3$ | NCHO | S | 2-F, 4-Cl | B1 |
| $CH_3$ | $CH_3$ | NCHO | S | 2-F, 4-$CH_3$ | B1 |
| $CH_3$ | $CH_3$ | NCHO | S | 3-F, 4-Cl | B1 |
| $CH_3$ | $CH_3$ | NCHO | S | 3-F, 4-$CH_3$ | B1 |
| $CH_3$ | $CH_3$ | NCHO | S | 2-Cl, 4-$CH_3$ | B1 |
| $CH_3$ | $CH_3$ | NCHO | S | 3-Cl, 4-$CH_3$ | B1 |
| $CH_3$ | $CH_3$ | NCHO | S | 2-Cl, 3-Cl, 4-Cl | B1 |
| $CH_3$ | $CH_3$ | $NCOCH_3$ | S | 2-F, 4-$CH_3$ | B1 |
| $CH_3$ | $CH_3$ | $NCOCH_3$ | S | 2-Cl, 3-Cl, 4-Cl | B1 |
| $CH_3$ | $CH_3$ | $NCH_3$ | S | 3-Cl, 4-$CH_3$ | B1 |

Table 1 (continued)

| $R^1$ | $R^2$ | X | Y | $W_n$ | B |
|---|---|---|---|---|---|
| $CH_3$ | $CH_3$ | NNO | S | 2-Cl, 4-Cl | B 1 |
| $CH_3$ | $CH_3$ | NNO | S | 3-Cl, 4-$CH_3$ | B 1 |
| $CH_3$ | $CH_3$ | $NNH_2$ | S | 4-Cl | B 1 |
| $CH_3$ | $CH_3$ | $NNH_2$ | S | 2-Cl, 4-Cl | B 1 |
| $CH_3$ | $CH_3$ | $NNH_2$ | S | 3-Cl, 4-$CH_3$ | B 1 |
| $CH_3$ | $CH_3$ | $NNH_2$ | S | 3-F, 4-Cl | B 1 |
| $CH_3$ | $CH_3$ | $NNH_2$ | S | 3-F, 4-$CH_3$ | B 1 |
| $CH_3$ | $CH_3$ | $NSO_2CH_3$ | S | 2-Cl, 4-Cl | B 1 |
| $CH_3$ | $CH_3$ | $NSO_2CH_3$ | S | 3-Cl, 4-$CH_3$ | B 1 |
| $CH_3$ | $CH_3$ | $NSO_2CH_3$ | S | 3-F, 4-$CH_3$ | B 1 |
| $CH_3$ | $CH_3$ | $NSO_2CH_3$ | S | 2-Cl, 3-Cl, 4-Cl | B 1 |
| $CH_3$ | $CH_3$ | $CH_2$ | S | 4-Cl | B 1 |
| $CH_3$ | $CH_3$ | $CH_2$ | S | 2-Cl, 4-Cl | B 1 |
| $CH_3$ | $CH_3$ | $CH_2$ | S | 2-Cl, 4-$CH_3$ | B 1 |
| $CH_3$ | $CH_3$ | $CH_2$ | S | 2-F, 4-$CH_3$ | B 1 |
| $CH_3$ | $CH_3$ | CH(OH) | S | 4-Cl | B 1 |
| $CH_3$ | $CH_3$ | CH(OH) | S | 2-Cl, 4-Cl | B 1 |
| $CH_3$ | $CH_3$ | CH(OH) | S | 2-Cl, 4-$CH_3$ | B 1 |
| $CH_3$ | $CH_3$ | CH(OH) | S | 3-Cl, 4-$CH_3$ | B 1 |
| $CH_3$ | $CH_3$ | CH(OH) | S | 2-F, 4-$CH_3$ | B 1 |

Table 1 (continued)

| $R^1$ | $R^2$ | X | Y | $W_n$ | B |
|---|---|---|---|---|---|
| $CH_3$ | $CH_3$ | CH(OH) | S | 3-F, 4-$CH_3$ | B 1 |
| $CH_3$ | $CH_3$ | CH($OCH_3$) | S | 4-Cl | B 1 |
| $CH_3$ | $CH_3$ | CH($OCH_3$) | S | 2-Cl, 4-Cl | B 1 |
| $CH_3$ | $CH_3$ | CH($OCOCH_3$) | S | 4-Cl | B 1 |
| $CH_3$ | $CH_3$ | CH($OCOCH_3$) | S | 2-Cl, 4-Cl | B 1 |
| $CH_3$ | $CH_3$ | CH($OCOCH_3$) | S | 2-Cl, 4-$CH_3$ | B 1 |
| $CH_3$ | $CH_3$ | CH($OCOCH_3$) | S | 2-F, 4-$CH_3$ | B 1 |
| $CH_3$ | $CH_3$ | CH(F) | S | 4-Cl | B 1 |
| $CH_3$ | $CH_3$ | CH(F) | S | 2-Cl, 4-Cl | B 1 |
| $CH_3$ | $CH_3$ | CH(Cl) | S | 4-Cl | B 1 |
| $CH_3$ | $CH_3$ | CH(Cl) | S | 2-Cl, 4-Cl | B 1 |
| $CH_3$ | $CH_3$ | C=O | S | 4-Cl | B 1 |
| $CH_3$ | $CH_3$ | C=O | S | 2-Cl, 4-Cl | B 1 |
| $CH_3$ | $CH_3$ | C=O | S | 2-Cl, 4-$CH_3$ | B 1 |
| $CH_3$ | $CH_3$ | C=O | S | 2-F, 4-$CH_3$ | B 1 |
| $CH_3$ | $CH_3$ | $CH_2$ | S | 4-Cl | B 2 |
| $CH_3$ | $CH_3$ | $CH_2$ | S | 2-Cl, 4-Cl | B 2 |
| $CH_3$ | $CH_3$ | $CH_2$ | S | 2-Cl, 4-$CH_3$ | B 2 |
| $CH_3$ | $CH_3$ | $CH_2$ | S | 2-F, 4-$CH_3$ | B 2 |
| $CH_3$ | $CH_3$ | CH(OH) | S | 4-Cl | B 2 |

Table 1 (continued)

| $R^1$ | $R^2$ | X | Y | $W_n$ | B |
|---|---|---|---|---|---|
| $CH_3$ | $CH_3$ | CH(OH) | S | 2-Cl, 4-Cl | B 2 |
| $CH_3$ | $CH_3$ | CH(OH) | S | 2-Cl, 4-$CH_3$ | B 2 |
| $CH_3$ | $CH_3$ | CH(OH) | S | 3-Cl, 4-$CH_3$ | B 2 |
| $CH_3$ | $CH_3$ | CH(OH) | S | 2-F, 4-$CH_3$ | B 2 |
| $CH_3$ | $CH_3$ | CH(OH) | S | 3-F, 4-$CH_3$ | B 2 |
| $CH_3$ | $CH_3$ | CH($OCH_3$) | S | 4-Cl | B 2 |
| $CH_3$ | $CH_3$ | CH($OCH_3$) | S | 2-Cl, 4-Cl | B 2 |
| $CH_3$ | $CH_3$ | CH($OCOCH_3$) | S | 4-Cl | B 2 |
| $CH_3$ | $CH_3$ | CH($OCOCH_3$) | S | 2-Cl, 4-Cl | B 2 |
| $CH_3$ | $CH_3$ | CH($OCOCH_3$) | S | 2-Cl, 4-$CH_3$ | B 2 |
| $CH_3$ | $CH_3$ | CH($OCOCH_3$) | S | 2-F, 4-$CH_3$ | B 2 |
| $CH_3$ | $CH_3$ | CH(F) | S | 4-Cl | B 2 |
| $CH_3$ | $CH_3$ | CH(F) | S | 2-Cl, 4-Cl | B 2 |
| $CH_3$ | $CH_3$ | CH(Cl) | S | 4-Cl | B 2 |
| $CH_3$ | $CH_3$ | CH(Cl) | S | 2-Cl, 4-Cl | B 2 |
| $CH_3$ | $CH_3$ | C=O | S | 4-Cl | B 2 |
| $CH_3$ | $CH_3$ | C=O | S | 2-Cl, 4-Cl | B 2 |
| $CH_3$ | $CH_3$ | C=O | S | 2-Cl, 4-$CH_3$ | B 2 |
| $CH_3$ | $CH_3$ | C=O | S | 2-F, 4-$CH_3$ | B 2 |
| $CH_3$ | $CH_3$ | C($CH_3$)(OH) | S | 4-Cl | B 2 |

Table 1 (continued)

| $R^1$ | $R^2$ | X | Y | $W_n$ | B |
|---|---|---|---|---|---|
| $CH_3$ | $CH_3$ | $C(CH_3)(OH)$ | S | 2-Cl, 4-Cl | B 2 |
| $CH_3$ | $CH_3$ | $C(CH_3)(OCOCH_3)$ | S | 4-Cl | B 2 |
| $CH_3$ | $CH_3$ | $C(CH_3)(OCOCH_3)$ | S | 2-Cl, 4-Cl | B 2 |
| $CH_3$ | $CH_3$ | $C(CH_3)(F)$ | S | 4-Cl | B 2 |
| $CH_3$ | $CH_3$ | $C(CH_3)(F)$ | S | 2-Cl, 4-Cl | B 2 |
| $CH_3$ | $CH_3$ | $C(C_2H_5)(OH)$ | S | 4-Cl | B 2 |
| $CH_3$ | $CH_3$ | $C(C_2H_5)(OH)$ | S | 2-Cl, 4-Cl | B 2 |
| $CH_3$ | $CH_3$ | $CH(CH_3)$ | S | 4-Cl | B 2 |
| $CH_3$ | $CH_3$ | $CH(CH_3)$ | S | 2-Cl, 4-Cl | B 2 |
| $CH_3$ | $CH_3$ | $CH(CH_3)$ | S | 2-Cl, 4-$CH_3$ | B 2 |
| $CH_3$ | $CH_3$ | $CH(CH_3)$ | S | 2-F, 4-$CH_3$ | B 2 |
| $CH_3$ | $CH_3$ | CH(OH) | O | 4-$CH_3$ | B 2 |
| $CH_3$ | $CH_3$ | CH(OH) | O | 2-Cl, 4-Cl | B 2 |
| $CH_3$ | $CH_3$ | $CH_2$ | O | 4-$CH_3$ | B 2 |
| $CH_3$ | $CH_3$ | $CH_2$ | O | 2-Cl, 4-Cl | B 2 |
| Cl | $CH_3$ | $CH_2$ | S | 4-Cl | B 2 |
| Cl | $CH_3$ | $CH_2$ | S | 2-Cl, 4-Cl | B 2 |
| Cl | $CH_3$ | $CH_2$ | S | 2-Cl, 4-$CH_3$ | B 2 |
| Cl | $CH_3$ | $CH_2$ | S | 2-F, 4-$CH_3$ | B 2 |
| $CH_3O$ | $CH_3$ | $CH_2$ | S | 4-Cl | B 2 |

Table 1 (continued)

| $R^1$ | $R^2$ | X | Y | $W_n$ | B |
|---|---|---|---|---|---|
| $CH_3O$ | $CH_3$ | $CH_2$ | S | 2-Cl, 4-Cl | B 2 |
| $CH_3O$ | $CH_3$ | $CH_2$ | S | 2-Cl, 4-$CH_3$ | B 2 |
| $CH_3O$ | $CH_3$ | $CH_2$ | S | 2-F, 4-$CH_3$ | B 2 |
| $CH_3$ | $CH_3$ | S | S | H | B 1 |
| $CH_3$ | $CH_3$ | S | S | 4-Cl | B 1 |
| $CH_3$ | $CH_3$ | S | S | 4-$CH_3$ | B 1 |
| $CH_3$ | $CH_3$ | S | S | 2-Cl, 4-Cl | B 1 |
| $CH_3$ | $CH_3$ | S | S | 3-F, 4-$CH_3$ | B 1 |
| $CH_3$ | $CH_3$ | S | S | 2-Cl, 4-$CH_3$ | B 1 |
| $CH_3$ | $CH_3$ | S | S | 3-Cl, 4-$CH_3$ | B 1 |
| $CH_3$ | $CH_3$ | S | S | 2-Cl, 3-Cl, 4-Cl | B 1 |
| $CH_3$ | $CH_3$ | S | O | 4-Cl | B 1 |
| $CH_3$ | $CH_3$ | S | O | 4-$CH_3$ | B 1 |
| $CH_3$ | $CH_3$ | S | O | 2-Cl, 4-Cl | B 1 |
| $CH_3$ | $CH_3$ | S | O | 3-F, 4-$CH_3$ | B 1 |
| $CH_3$ | $CH_3$ | S | O | 2-Cl, 4-$CH_3$ | B 1 |
| $CH_3$ | $CH_3$ | S | NH | 4-Cl | B 1 |
| $CH_3$ | $CH_3$ | S | NH | 2-Cl, 4-Cl | B 1 |
| $CH_3$ | $CH_3$ | S | NH | 3-Cl, 4-$CH_3$ | B 1 |
| $CH_3$ | $CH_3$ | S | NCHO | 4-Cl | B 1 |

Table 1 (continued)

| $R^1$ | $R^2$ | X | Y | $W_n$ | B |
|---|---|---|---|---|---|
| $CH_3$ | $CH_3$ | S | NCHO | 2-Cl, 4-Cl | B 1 |
| $CH_3$ | $CH_3$ | S | NCHO | 3-Cl, 4-$CH_3$ | B 1 |
| $CH_3$ | $CH_3$ | S | S | 2-Cl, 4-Cl | B 2 |
| $CH_3$ | $CH_3$ | S | O | 2-Cl, 4-Cl | B 2 |
| $CH_3$ | $CH_3$ | S | NH | 4-Cl | B 2 |
| $CH_3$ | $CH_3$ | S | NH | 2-Cl, 4-Cl | B 2 |
| $CH_3$ | $CH_3$ | S | NCHO | 4-Cl | B 2 |
| $CH_3$ | $CH_3$ | O | S | H | B 1 |
| $CH_3$ | $CH_3$ | O | S | 4-Cl | B 1 |
| $CH_3$ | $CH_3$ | O | S | 4-$CH_3$ | B 1 |
| $CH_3$ | $CH_3$ | O | S | 2-Cl, 4-Cl | B 1 |
| $CH_3$ | $CH_3$ | O | S | 3-F, 4-$CH_3$ | B 1 |
| $CH_3$ | $CH_3$ | O | S | 2-Cl, 4-$CH_3$ | B 1 |
| $CH_3$ | $CH_3$ | O | S | 3-Cl, 4-$CH_3$ | B 1 |
| $CH_3$ | $CH_3$ | O | S | 2-Cl, 3-Cl, 4-Cl | B 1 |
| $CH_3$ | $CH_3$ | O | O | 4-Cl | B 1 |
| $CH_3$ | $CH_3$ | O | O | 4-$CH_3$ | B 1 |
| $CH_3$ | $CH_3$ | O | O | 2-Cl, 4-Cl | B 1 |
| $CH_3$ | $CH_3$ | O | O | 3-F, 4-$CH_3$ | B 1 |
| $CH_3$ | $CH_3$ | O | O | 2-Cl, 4-$CH_3$ | B 1 |

Table 1 (continued)

| $R^1$ | $R^2$ | X | Y | $W_n$ | B |
|---|---|---|---|---|---|
| $CH_3$ | $CH_3$ | O | NH | 4-Cl | B 1 |
| $CH_3$ | $CH_3$ | O | NH | 2-Cl, 4-Cl | B 1 |
| $CH_3$ | $CH_3$ | O | NH | 3-Cl, 4-$CH_3$ | B 1 |
| $CH_3$ | $CH_3$ | O | NCHO | 4-Cl | B 1 |
| $CH_3$ | $CH_3$ | O | NCHO | 2-Cl, 4-Cl | B 1 |
| $CH_3$ | $CH_3$ | O | NCHO | 3-Cl, 4-$CH_3$ | B 1 |
| $CH_3$ | $CH_3$ | O | SO | 4-Cl | B 1 |
| $CH_3$ | $CH_3$ | O | SO | 2-Cl, 4-Cl | B 1 |
| $CH_3$ | $CH_3$ | O | $SO_2$ | 4-Cl | B 1 |
| $CH_3$ | $CH_3$ | O | $SO_2$ | 2-Cl, 4-Cl | B 1 |
| $CH_3$ | $CH_3$ | O | $SO_2$ | 3-Cl, 4-$CH_3$ | B 1 |
| $CH_3$ | $CH_3$ | O | S | H | B 2 |
| $CH_3$ | $CH_3$ | O | S | 4-Cl | B 2 |
| $CH_3$ | $CH_3$ | O | S | 4-$CH_3$ | B 2 |
| $CH_3$ | $CH_3$ | O | S | 2-Cl, 4-Cl | B 2 |
| $CH_3$ | $CH_3$ | O | S | 3-F, 4-$CH_3$ | B 2 |
| $CH_3$ | $CH_3$ | O | S | 2-Cl, 4-$CH_3$ | B 2 |
| $CH_3$ | $CH_3$ | O | S | 3-Cl, 4-$CH_3$ | B 2 |
| $CH_3$ | $CH_3$ | O | S | 2-Cl, 3-Cl, 4-Cl | B 2 |
| $CH_3$ | $CH_3$ | O | O | 4-Cl | B 2 |

Table 1 (continued)

| $R^1$ | $R^2$ | X | Y | $W_n$ | B |
|---|---|---|---|---|---|
| $CH_3$ | $CH_3$ | O | O | 4-$CH_3$ | B 2 |
| $CH_3$ | $CH_3$ | O | O | 2-Cl, 4-Cl | B 2 |
| $CH_3$ | $CH_3$ | O | O | 3-F, 4-$CH_3$ | B 2 |
| $CH_3$ | $CH_3$ | O | O | 2-Cl, 4-$CH_3$ | B 2 |
| $CH_3$ | $CH_3$ | O | NH | 4-Cl | B 2 |
| $CH_3$ | $CH_3$ | O | NH | 2-Cl, 4-Cl | B 2 |
| $CH_3$ | $CH_3$ | O | NH | 3-Cl, 4-$CH_3$ | B 2 |
| $CH_3$ | $CH_3$ | O | NCHO | 4-Cl | B 2 |
| $CH_3$ | $CH_3$ | O | NCHO | 2-Cl, 4-Cl | B 2 |
| $CH_3$ | $CH_3$ | O | NCHO | 3-Cl, 4-$CH_3$ | B 2 |
| $CH_3$ | $CH_3$ | O | SO | 4-Cl | B 2 |
| $CH_3$ | $CH_3$ | O | SO | 2-Cl, 4-Cl | B 2 |
| $CH_3$ | $CH_3$ | O | $SO_2$ | 4-Cl | B 2 |
| $CH_3$ | $CH_3$ | O | $SO_2$ | 2-Cl, 4-Cl | B 2 |
| $CH_3$ | $CH_3$ | O | $SO_2$ | 3-Cl, 4-$CH_3$ | B 2 |
| Cl | $CH_3$ | O | S | H | B 1 |
| Cl | $CH_3$ | O | S | 4-Cl | B 1 |
| Cl | $CH_3$ | O | S | 4-$CH_3$ | B 1 |
| Cl | $CH_3$ | O | S | 2-Cl, 4-Cl | B 1 |
| Cl | $CH_3$ | O | S | 3-F, 4-$CH_3$ | B 1 |

Table 1 (continued)

| $R^1$ | $R^2$ | X | Y | $W_n$ | B |
|---|---|---|---|---|---|
| Cl | $CH_3$ | O | S | 2-Cl, 4-$CH_3$ | B 1 |
| Cl | $CH_3$ | O | S | 3-Cl, 4-$CH_3$ | B 1 |
| Cl | $CH_3$ | O | S | 2-Cl, 3-Cl, 4-Cl | B 1 |
| Cl | $CH_3$ | O | S | 2-Cl, 4-Cl | B 2 |
| Cl | $CH_3$ | O | S | 3-F, 4-$CH_3$ | B 2 |
| Cl | $CH_3$ | O | S | 2-Cl, 4-$CH_3$ | B 2 |
| Cl | $CH_3$ | O | S | 3-Cl, 4-$CH_3$ | B 2 |
| Cl | $CH_3$ | O | S | 2-Cl, 4-$CH_3$ | B 2 |
| Cl | $CH_3$ | O | S | 2-Cl, 4-Cl | B 3 |
| Cl | $CH_3$ | O | S | 2-Cl, 4-Cl | B 4 |
| Cl | $CH_3$ | O | S | 2-Cl, 4-Cl | B 5 |
| Cl | $CH_3$ | O | S | 2-Cl, 4-Cl | B 6 |
| Cl | $CH_3$ | O | S | 2-Cl, 4-Cl | B 8 |
| Cl | $CH_3$ | O | S | 2-Cl, 4-Cl | B 13 |
| Cl | $CH_3$ | O | S | 2-Cl, 4-Cl | B 14 |
| Cl | $CH_3$ | O | S | 2-Cl, 4-Cl | B 15 |
| Cl | $CH_3$ | O | S | 2-Cl, 4-Cl | B 16 |
| Cl | $CH_3$ | O | S | 2-Cl, 4-Cl | B 17 |
| Cl | $CH_3$ | O | S | 2-Cl, 4-Cl | B 18 |

In the above-mentioned table, B1 to B18 represent the following chemical structures:

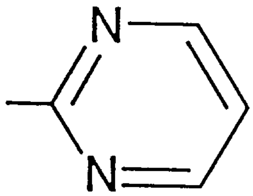
B1
,
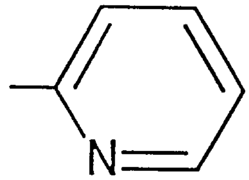
B2
,
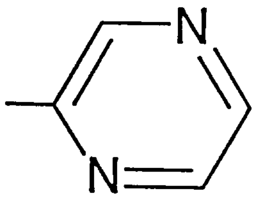
B3
,
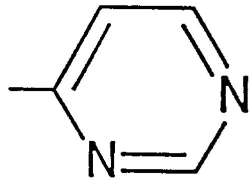
B4
,
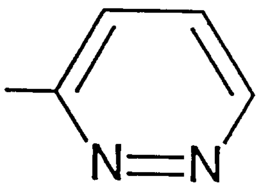
B5
,
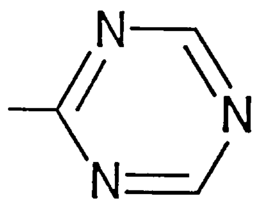
B6
,

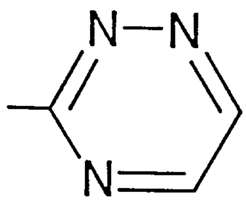
B7 ,

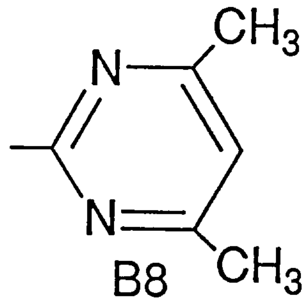
B8 ,

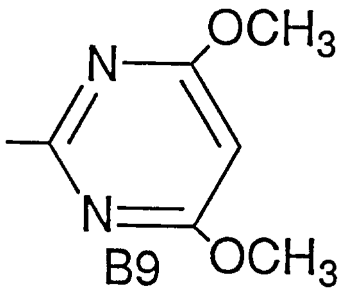
B9 ,

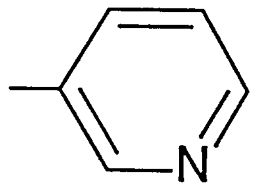
B10 ,

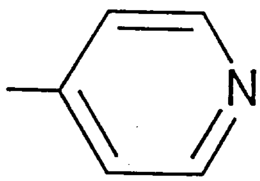
B11 ,

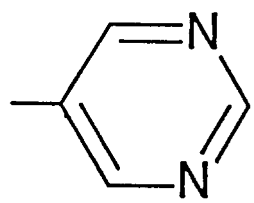
B12 ,

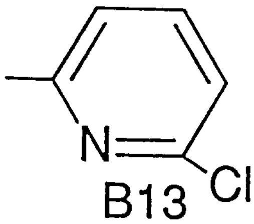
B13 ,

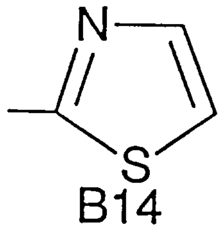
B14 ,

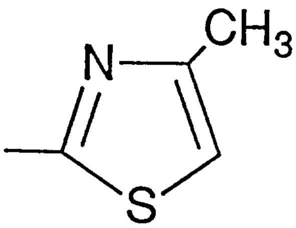
B15 ,

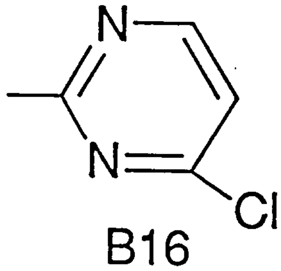
B16 ,

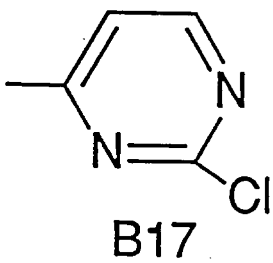
B17 ,

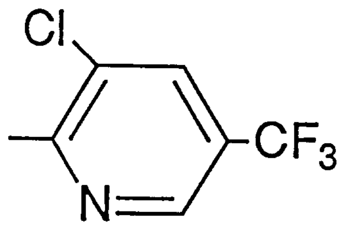
B18 ,

Next, methods for producing the compounds of the present invention will be explained below.

(1) A substituted pyrazole derivative of a general formula [4]:

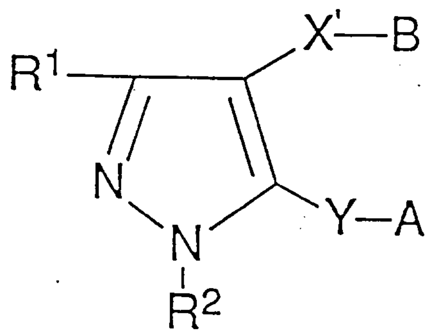
[4]

wherein $R^1$ represents a hydrogen atom, a halogen atom, an alkyl group, an alkoxy group, an alkylthio group, a haloalkyl group, a cyano group, an alkoxycarbonyl group, or an unsubstituted or substituted phenyl group;

$R^2$ represents a hydrogen atom, an alkyl group, a haloalkyl group, an unsubstituted or substituted phenyl group, an unsubstituted or substituted phenylalkyl group, $-COR^6$, or $-SO_2R^7$;

X' represents an oxygen atom, -S-, or $-N(R^3)-$;

Y represents an oxygen atom, -S-, -SO-, $-SO_2-$, or $-N(R^3)-$;

$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, an unsubstituted or substituted phenylalkyl group, $-COR^6$, or $-SO_2R^7$;

$R^6$ represents a hydrogen atom, an alkyl group, a haloalkyl group, an unsubstituted or substituted phenyl group, an unsubstituted or substituted phenylalkyl group, or an alkoxy group;
$R^7$ represents an alkyl group, a haloalkyl group, or an unsubstituted or substituted phenyl group;
A represents an unsubstituted or substituted phenyl group; and
B represents an unsubstituted or substituted heterocyclic group,
is produced, by reacting a substituted pyrazole of formula [2]:

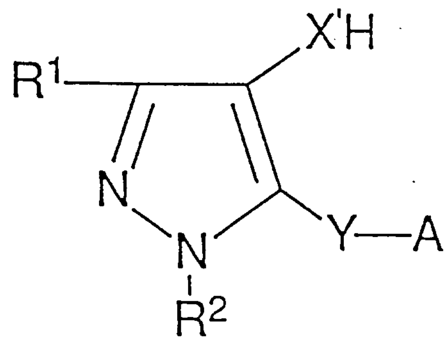

[2]

wherein $R^1$, $R^2$, Y and A have the same meanings as those mentioned above; X' represents an oxygen atom, -S- or -N($R^3$)-, and $R^3$ has the same meaning as that mentioned above,
and a heterocyclic compound of a general formula [3]:

L-B [3]

wherein L represents a split-off group such as a halogen atom, etc.; and B represents an unsubstituted or substituted heterocyclic group. In this process, when X' in formula [2] is $-NCOR^6$ or $-NSO_2R^7$, the product is hydrolyzed, as the case may be, by post-treatment to give a compound of formula [4] where X' is -NH. This reaction does not always require a solvent. If used, however, the solvent for the reaction includes, for example, hydrocarbons such as toluene, xylene, etc., halogenated hydrocarbons such as chlorobenzene, dichloroethane, etc., ethers such as diisopropyl ether, dioxane, etc., esters such as ethyl acetate, etc., nitriles such as acetonitrile, etc., and polar solvents such as dimethylsulfoxide, dimethylformamide, etc.

If desired, an organic base (e.g., pyridine, triethylamine, etc.) and an inorganic base (e.g., potassium carbonate, sodium hydride, etc.) may be added to the reaction system.

Also if desired, a copper salt and a copper complex may be added thereto as a catalyst.

The amounts of the reactants to be used in the above-mentioned reaction are such that the heterocyclic compound of formula [3] is used in an amount of from 1 to 5 equivalents relative to one equivalent of the substituted pyrazole of formula [2].

The reaction temperature in carrying out the above-mentioned reaction is not limited but, in general, it is preferably within the range of from room temperature to 200 °C or to the refluxing temperature of the solvent used.

After the reaction, the reaction mixture is subjected to ordinary post-treatment to obtain the intended product.

(2) To obtain compounds of formula [1] where X is -CO- or $-C(R^4)(R^5)-$, a substituted pyrazole of a general formula [6]:

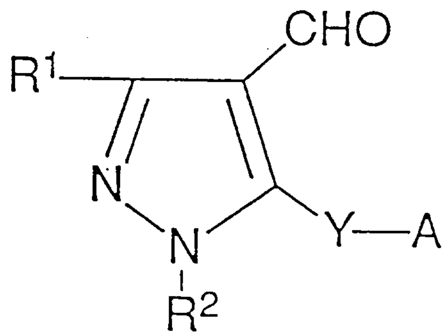

[6]

wherein $R^1$, $R^2$, Y and A have the same meanings as those mentioned above, is reacted with an organic metal compound of a general formula [7]:

Me-B [7]

wherein B has the same meaning as that mentioned above, and Met represents a metal such as Li, MgBr or the like, to give a substituted pyrazole of a general formula [8]:

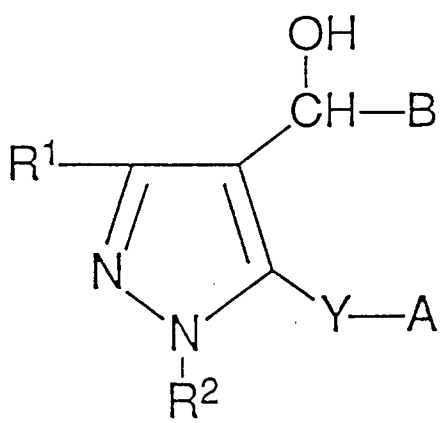

[8]

wherein $R^1$, $R^2$, Y, A and B have the same meanings as those mentioned above.

The compound of formula [8] is oxidized with a suitable oxidizing agent to give a substituted pyrazole of a general formula [9]:

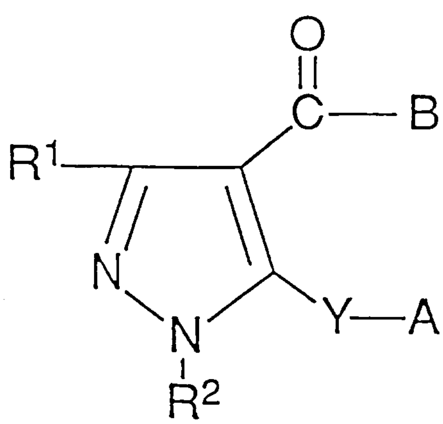

[9]

wherein $R^1$, $R^2$, Y, A and B have the same meanings as those mentioned above.

Alternatively, the compound of formula [8] is reduced with a suitable reducing agent to give a substituted pyrazole of a general formula [10]:

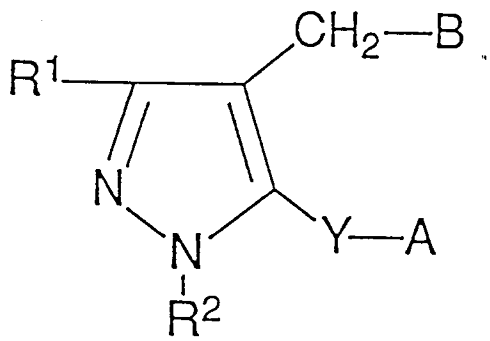

[10]

wherein $R^1$, $R^2$, Y, A and B have the same meanings as those mentioned above.

(3) To obtain compounds of formula [1] where X is -S-, a substituted pyrazole of a general formula [11]:

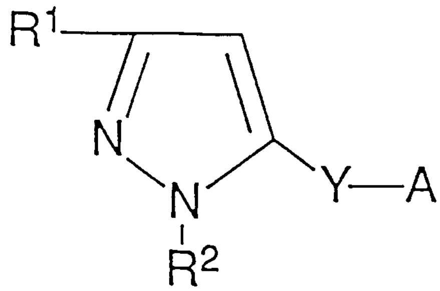

[11]

wherein $R^1$, $R^2$, Y and A have the same meanings as those mentioned above, is reacted with a sulfenyl chloride of a general formula [12]:

Cl-S-B [12]

wherein B has the same meaning as that mentioned above, to give a compound of the present invention of a general formula [13]:

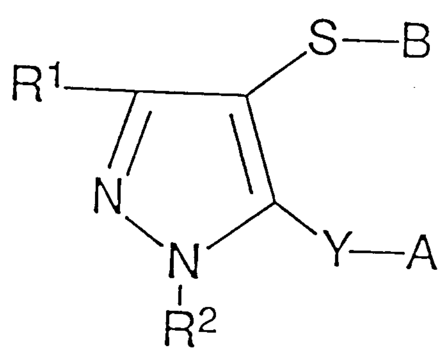

[13]

wherein $R^1$, $R^2$, Y, A and B have the same meanings as those mentioned above.

Substituted pyrazole derivatives of the following general formula [5], which are intermediates to be used for producing compounds of formula [1], and their salts are also novel compounds. The present invention is also directed to these.

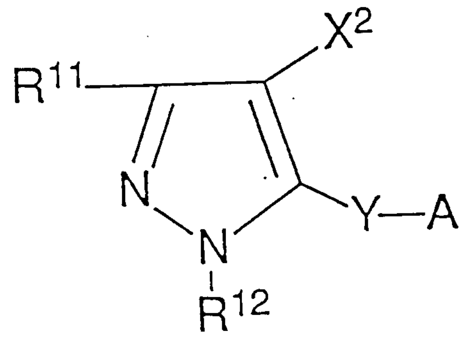

[5]

wherein $R^{11}$ represents a hydrogen atom, a halogen atom, an alkyl group, an alkoxy group, an alkylthio group, a haloalkyl group, a cyano group, an alkoxycarbonyl group, or an unsubstituted or substituted phenyl group;
$R^{12}$ represents a hydrogen atom, an alkyl group, a haloalkyl group, an unsubsituted or substituted phenyl group, an unsubstituted or substituted phenylalkyl group, $-COR^6$, or $-SO_2R^7$;
Y represents an oxygen atom, -S-, -SO-, $SO_2$-, or $-N(R^3)$-;
$X^2$ represents a nitro group, $-NH(R^3)$, -OH, -SH, -CHO, an alkoxycarbonyl group, or $-NHC(=S)NH_2$;
$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, a substituted or unsubstituted phenylalkyl group, $-COR^6$, or $-SO_2R^7$;
$R^6$ represents a hydrogen atom, an alkyl group, a haloalkyl group, a substituted or unsubstituted phenyl group, a substituted or unsubstituted phenylalkyl group, an alkoxy group, or $-N(R^9)(R^{10})$;
$R^7$ represents an alkyl group, a haloalkyl group, an unsubstituted or substituted phenyl group, or $-N(R^9)(R^{10})$;
$R^9$ and $R^{10}$ each independently represent a hydrogen atom, an alkyl group, or an unsubstituted or substituted phenyl group;
A represents an unsubstituted or substituted phenyl group.

Of compounds of formula [5], preferred are those where Y is -S- or an oxygen atom.

More preferred are those of formula [5] where $R^{11}$ is a halogen atom or an alkyl group, $R^{12}$ is an alkyl group, $X^2$ is a nitro group, $-NH(R^3)$ or -OH, and Y is -S-or an oxygen atom.

EMBODIMENTS OF THE PRESENT INVENTION

Next, concrete production examples will be shown below.

Example 1:

Production of 5-(2,4-dichlorophenylthio)-1,3-dimethyl-4-nitropyrazole:

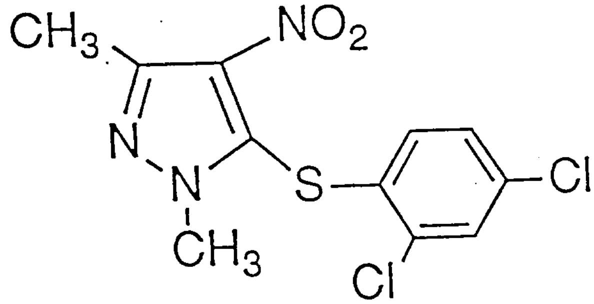

1.3 g of sodium hydride (55 %) and 5 g of sodium hydrosulfite were suspended in 50 ml of DMF, and 6 ml of a DMF solution containing 5 g (28 mmol) of 2,4-dichlorothiophenol dissolved therein were dropwise

added thereto while cooling with ice water. This was stirred for one hour while cooling with ice water, and 4.5 g (25 mmol) of 5-chloro-1,3-dimethyl-4-nitropyrazole were added thereto and stirred for 15 hours at room temperature. The solvent was removed by distillation, and 50 ml of water were added to the residue, which was then extracted two times each with 100 ml of chloroform. The organic layer was washed with 50 ml of aqueous 10 % sodium hydroxide solution, then with water, and dried with anhydrous sodium sulfate. After this was filtered, the chloroform was removed therefrom by distillation under reduced pressure. The residue was washed with n-hexane to obtain 6.25 g of the intended product 5-(2,4-dichlorophenylthio)-1,3-dimethyl-4-nitropyrazole as pale yellow crystals. The yield of the product was 79 %.

Example 2:

Production of 4-amino-5-(2,4-dichlorophenylthio)-1,3-dimethylpyrazole (Compound No. 142 of the invention):

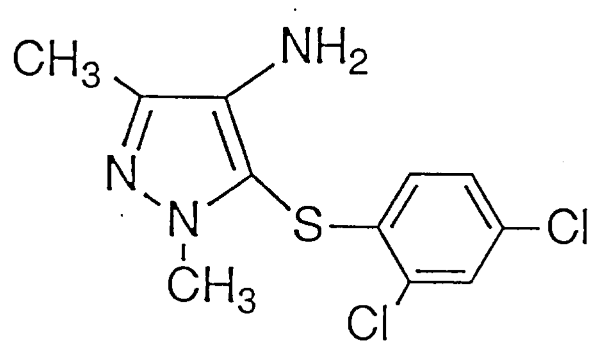

3 g (9.5 mmol) of 5-(2,4-dichlorphenylthio)-1,3-dimethyl-4-nitropyrazole were dissolved in 60 ml of methanol. 0.1 g of platinum dioxide was added thereto, and hydrogen gas was introduced thereinto under normal pressure to hydrogenate the compound. After the absorption of a theoretical amount of hydrogen was confirmed, the reaction was stopped and the catalyst was removed by filtration through Celite. The solvent was removed by filtration under reduced pressure, and the residue was dissolved in 50 ml of chloroform and dried with anhydrous sodium sulfate. After this was filtered, the chloroform was removed therefrom by distillation to obtain 2.38 g of the intended product 4-amino-5-(2,4-dichlorophenylthio)-1,3-dimethylpyrazole as a brown liquid. Its yield was 90 %.

Example 3:

Production of N-(5-(2,4-dichlorophenylthio)-1,3-dimethyl-4-pyrazolyl)formamide (Compound No. 143 of the invention):

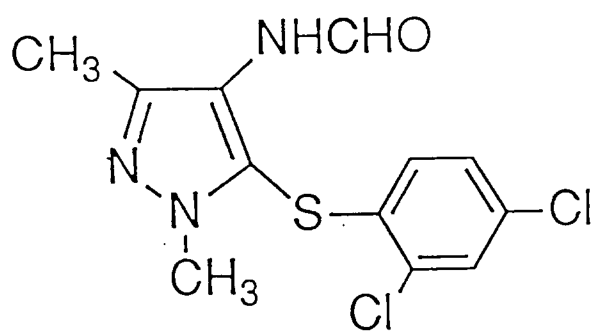

2 g of formic acid and 4.4 g of acetic anhydride were stirred and mixed at 50 °C for one hour. The resulting solution was cooled to 0 °C with ice water. To this, dropwise added was a mixed solution comprising 2.36 g of 4-amino-5-(2,4-dichlorophenylthio)-1,3-dimethylpyrazole (8.5 mmol) and 6.1 g of pyridine. This was stirred for 2 days at room temperature, then the solvent was removed therefrom by distillation under reduced pressure, and the residue was extracted with 100 ml of chloroform, washed with

water and dried with anhydrous sodium sulfate. After this was filtered, the chloroform was removed therefrom by distillation to obtain 2.3 g of the intended product N-(5-(2,4-dichlorophenylthio)-1,3-dimethyl-4-pyrazolyl)formamide as crystals. Its yield was 85 %. The compound had a melting point of 124.0 to 127.0 °C.

Example 4:

Production of 5-(2,4-dichlorophenylthio)-1,3-dimethyl-4-(2-pyridylamino)pyrazole (Compound No. 7 of the invention):

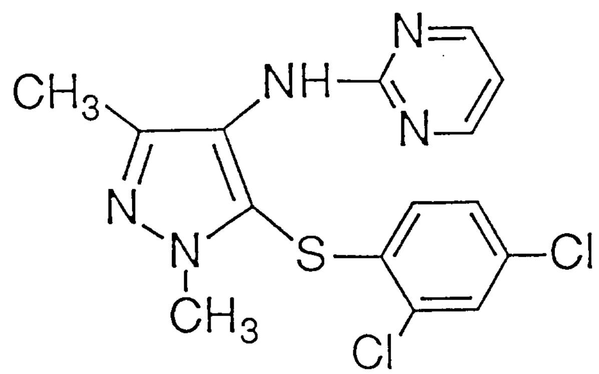

100 ml of a DMF solution containing 0.38 g of sodium hydride (55 %) suspended therein were cooled with ice water, and a solution prepared by dissolving 2.3 g (7.2 mmol) of N-(5-(2,4-dichlorophenylthio)-1,3-dimethyl-4-pyrazolyl)formamide in 20 ml of DMF was dropwise added thereto. This was stirred for one hour at room temperature, and 1.1 g (9.6 mmol) of 2-chloropyrimidine were added thereto and stirred under heat at 120 °C for 18 hours. After this was cooled, the solvent was removed therefrom by distillation, and the residue was extracted with 50 ml of chloroform, washed with water and dried with anhydrous sodium sulfate. After filtered and concentrated, the residue was purified by silica gel column chromatography (developer: chloroform) to obtain 1.0 g of Compound No. 7 of the invention. Its yield was 37 %. This had a melting point of 155.0 to 157.0 °C.

$^{1}H$ - NMR δ (ppm, $CDCl_8$)

2.25 (3H, s)、3.80 (3H, s)、6.57 (1H, t, J = 5Hz)、6.67 (1H, d, J = 9Hz)、7.01 (1H, dd, J = 2Hz, 9Hz)、7.29 (1H, d, J = 2Hz)、7.81 (1H, bs)、8.23 (2H, d, J = 5Hz)

Example 5:

Production of 5-(2,4-dichlorophenylthio)-1,3-dimethyl-4-(N-(2-pyrimidyl)-N-formylaminopyrazole (Compound No. 21 of the invention):

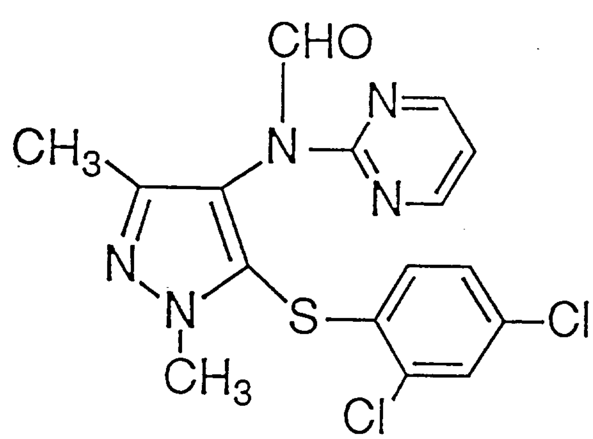

0.47 g (1.2 mmol) of 5-(2,4-dichlorophenylthio)-1,3-dimethyl-4-(2-pyrimidylamino)pyrazole were dissolved in a mixture comprising 1.2 g of formic acid and 2.6 g of acetic anhydride. While cooling with ice water, 3.7 g of pyridine were dropwise added thereto and stirred at room temperature for 3 days. The solvent was removed by distillation under reduced pressure, and water was added to the residue, which was then extracted with 50 ml of chloroform, washed with water and dried with anhydrous sodium sulfate. After this was filtered, the chloroform was removed therefrom by distillation to obtain 0.44 g of Compound No. 21 of the invention. Its yield was 87 %. The compound was a vitreous substance.
$^1$H-NMR δ (ppm, $CDCl_8$)
2.14 (3H, s)、3.83 (3H, s)、6.85 (1H, d, J = 9Hz)、7.09 (1H, t, J = 6Hz)、7.13 (1H, dd, J = 2Hz, 9Hz)、7.32 (1H, d, J = 2Hz)、8.54 (2H, d, J = 5Hz)、10.01 (1H, s)

Example 6:

Production of 3-chloro-5-(2,4-dichlorophenylthio)-1-methyl-4-nitropyrazole (Compound No. 110 of the invention):

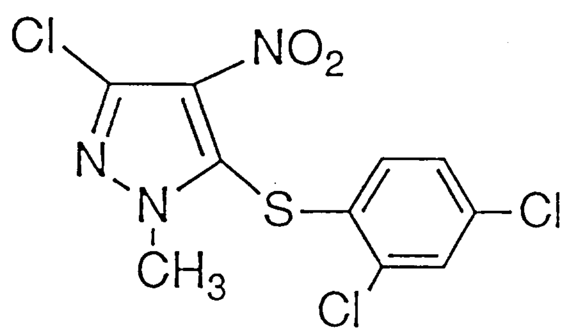

2.3 g (56 mmol) of sodium hydride (55 %) and 9.8 g (56 mmol) of sodium hydrosulfite were suspended in 80 ml of DMF, and 15 ml of a DMF solution containing 10.0 g (56 mmol) of 2,4-dichlorothiophenol dissolved therein were dropwise added thereto while cooling with ice water. After this was stirred for one hour while cooling with ice water, 40 ml of a DMF solution containing 10.0 g (51 mmol) of 3,5-dichloro-1-methyl-4-nitropyrazole were dropwise added thereto. Afterwards, this was stirred for 15 hours at room temperature. After the solvent was removed from this by distillation, the residue was dissolved in 200 ml of chloroform and washed with water. The chloroform layer was separated and dried on anhydrous sodium sulfate. The solvent was removed by distillation, and the residue was washed with isopropyl ether and normal hexane in this order to obtain 7.8 g of 3-chloro-5-(2,4-dichlorophenylthio)-1-methyl-4-nitropyrazole as white crystals. This had a melting point of 106 to 108°C.

Example 7:

Production of 4-amino-3-chloro-5-(2,4-dichlorothiophenyl)-1-methylpyrazole (Compound No. 123 of the invention):

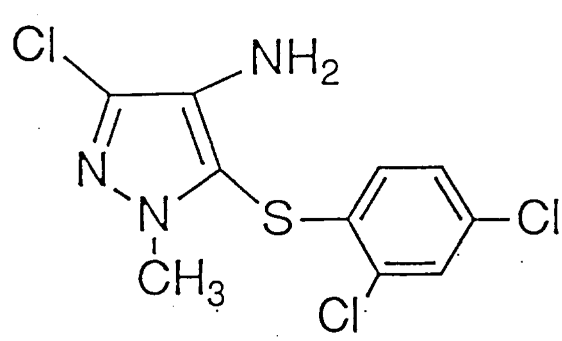

6.5 g (19.2 mmol) of 3-chloro-5-(2,4-dichlorophenylthio)-1-methyl-4-nitropyrazole were dissolved in 20 ml of ethanol, and 20 ml of concentrated hydrochloric acid were added thereto and stirred. Next, 20 ml of an ethanol solution containing 14.4 g (57.6 mmol) of stannous chloride were dropwise added thereto, heated up to 80 ° C and stirred for 3 hours. After left to be cooled to room temperature, this was adjusted to have pH of from 9 to 10 with aqueous 20 % sodium hydroxide solution while cooling on an ice bath. This was then extracted three times each with 100 ml of ethyl acetate. The ethyl acetate layers were combined, washed with saturated saline solution and dried on anhydrous sodium sulfate. The solvent was removed from this to obtain 4.4 g of 4-amino-3-chloro-5-(2,4-dichlorophenylthio)-1-methylpyrazole as white crystals. The compound had a melting point of 86 to 87 ° C.

Example 8:

Production of N-(3-chloro-5-(2,4-dichlorophenylthio)-1-methyl-4-pyrazolyl)formamide (Compound No. 132 of the invention):

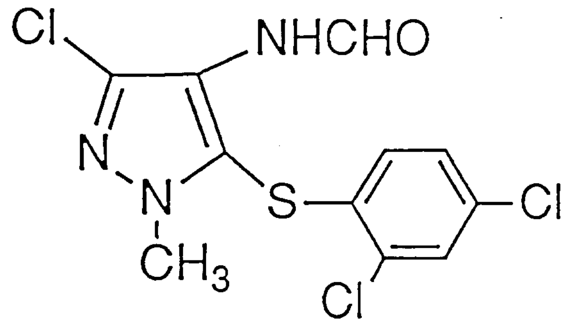

3.0 g (64.8 mmol) of formic acid and 6.6 g (64.8 mmol) of acetic anhydride were stirred at 50 ° C for one hour. After this was left to be cooled to room temperature, 10.2 g (130 mmol) of a pyridine solution containing 4.0 g (13.0 mmol) of 4-amino-3-chloro-5-(2,4-dichlorophenylthio)-1-methylpyrazole were gradually added thereto and stirred for 2 days at room temperature. After the solvent was removed from this by distillation, the residue was extracted with 200 ml of chloroform and washed with water, and the chloroform layer was dried on anhydrous sodium sulfate. The solvent was removed from this by distillation to obtain 3.4 g of N-(3-chloro-5-(2,4-dichlorophenylthio)-1-methyl-4-pyrazolyl)formamide as white crystals. Its yield was 77 %. The compound had a melting point of 166 to 167 ° C.

Example 9:

Production of 3-chloro-5-(2,4-dichlorophenylthio)-1-methyl-4-(2-pyridylamino)pyrazole (Compound No. 34 of the invention):

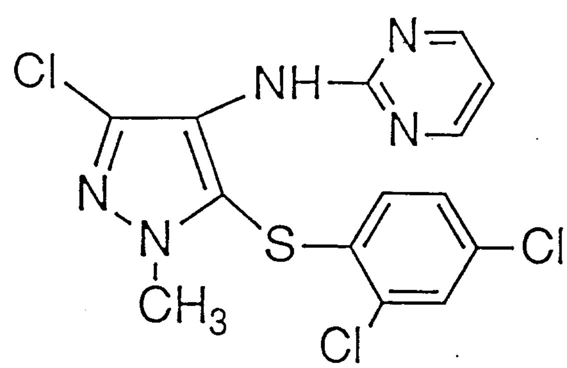

0.4 g (9.8 mmol) of sodium hydride (55 %) were suspended in 20 ml of DMF, and 30 ml of a DMF solution containing 3.0 g (8.9 mmol) of N-(3-chloro-5-(2,4-dichlorophenylthio)-1-methyl-4-pyrazolyl)-formamide dissolved therein were dropwise added thereto while cooling with ice water and stirred at room temperature for 2 hours. Next, 1.0 g (8.9 mmol) of 2-chloropyrimidine was added to the resulting solution, heated up to 120 °C and stirred for 15 hours. The solvent was removed by distillation under reduced pressure, the residue was dissolved in 200 ml of chloroform added thereto, washed with water and then dried with anhydrous sodium sulfate. The solvent was removed by distillation, and the residue was washed with isopropyl ether to obtain 0.9 g of 3-chloro-5-(2,4-dichlorophenylthio)-1-methyl-4-(2-pyrimidylamino)-pyrazole (Compound No. 34 of the invention) as white crystals. Its yield was 26 %. The compound had a melting point of 177 to 178 °C.

Example 10:

Production of 5-(2,4-dichlorophenylthio)-1,3-dimethyl-4-(2-thiazolylamino)pyrazole (Compound No. 32 of the invention):

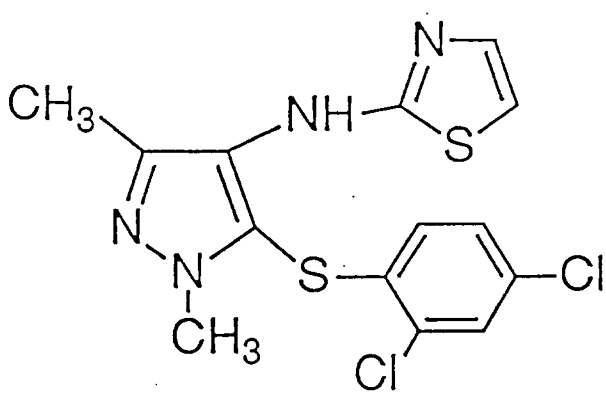

3.7 g of 4-amino-5-(2,4-dichlorophenylthio)-1,3-dimethylpyrazole were dissolved in a mixed solution comprising 50 ml of benzene and 50 ml of tetrahydrofuran, and 2.1 g of benzoylisothiocyanate were dropwise added thereto and stirred for 5 hours under heat to reflux. The reaction liquid was cooled, and the solvent was removed therefrom by distillation under reduced pressure. To the residue, added were 80 ml of an aqueous solution containing 1.1 g of sodium hydroxide and stirred for 2 hours under heat to reflux. After cooled, this was made acidic to have pH of about 4 by adding 3 N-hydrochloric acid thereto, whereupon crystals precipitated. The crystals were taken out by filtration, 50 ml of aqueous 28 % ammonia were added thereto and stirred at room temperature for 2 hours. Afterwards, the crystals were taken out by filtration, washed with water and dried to obtain 4.1 g of N-(5-(2,4-dichlorophenylthio)-1,3-dimethyl-4-pyrazolyl)-thiourea.

1.0 g of this thiourea derivative and 1.5 g of aqueous 40 % chloroacetaldehyde solution were added to 30 ml of ethanol and stirred for 4 hours under heat to reflux. After this was cooled, the solvent was removed therefrom by distillation under reduced pressure, and an aqueous sodium carbonate solution was added to the residue. After stirred for a little while, this was extracted with chloroform and dried with anhydrous sodium sulfate. After this was filtered, the chloroform was removed therefrom by distillation under reduced pressure to give crystals. These crystals were washed with isopropyl ether and dried to obtain 0.9 g of 5-(2,4-dichlorophenylthio)-1,3-dimethyl-4-(2-thiazolylamino)pyrazole (Compound No. 32 of the invention). This had a melting point of 153 to 155 °C.

Example 11:

Production of 5-(2,4-dichlorophenylthio)-1,3-dimethyl-4-(2-pyridylamino)pyrazole (Compound No. 29 of the invention):

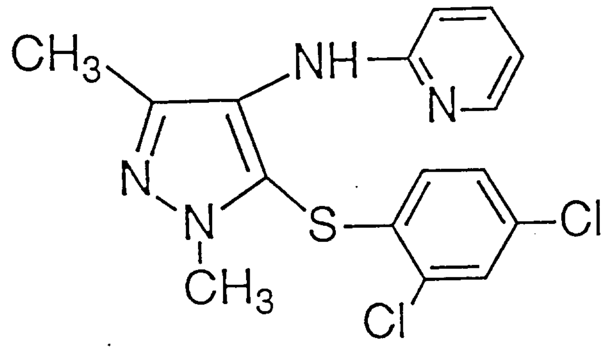

80 ml of dimethylformamide were added to a mixture comprising 1.0 g of N-[5-(2,4-dichlorophenylthio)-1,3-dimethyl-4-pyrazolyl]formamide, 0.44 g of anhydrous potassium carbonate, 0.4 g of copper(I) bromide and 0.5 g of 2-bromopyridine and stirred for 5 hours under heat to reflux. After this was cooled, the solvent was removed by distillation under reduced pressure, and water was added to the residue, which was then extracted with chloroform. The organic layer was dried with anhydrous sodium sulfate. After this was filtered, the chloroform was removed therefrom by distillation under reduced pressure, and the residue was purified by silica gel column chromatography (developer: chloroform) to obtain 0.32 g of 5-(2,4-dichlorophenylthio)-1,3-dimethyl-4-(2-pyridylamino)pyrazole (Compound No. 29 of the invention). This had a melting point of 135 to 136 ° C.

Example 12:

Production of 5-(4-chlorophenylamino)-1,3-dimethyl-4-(2-pyrimidylthio)pyrazole (Compound No. 45 of the invention):

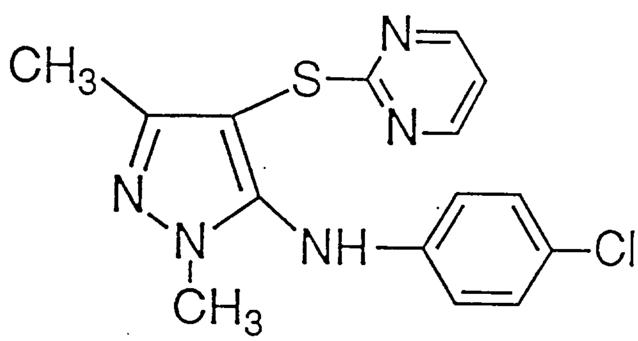

2.0 g (9.0 mmol) of 5-(4-chlorophenylamino)-1,3-dimethylpyrazole were dissolved in 50 ml of chloroform, and 1.7 g (11.7 mmol) of 2-pyrimidylsulfenyl chloride were dropwise added to the resulting solution at room temperature and thereafter stirred for 4 hours still at room temperature. The reaction solution was adjusted to have pH of from 7 to 8, by adding aqueous, saturated sodium hydrogencarbonate solution thereto. The thus-separated aqueous layer was extracted three times each with 50 ml of chloroform. The chloroform layers were combined and dried with anhydrous sodium sulfate. The solvent was removed from this by distillation, and the residue was purified by silica gel column chromatography (developer: chloroform) to obtain 1.7 g of 5-(4-chlorophenylamino)-1,3-dimethyl-4-(2-pyrimidylthio)pyrazole (Compound No. 45 of the invention) as white crystals. Its yield was 57 %. The compound had a melting point of 139 to 140 ° C.

Example 13:

Production of (5-(2,4-dichlorophenylthio)-1,3-dimethyl-4-pyrazolyl)-2-pyridylmethanol (Compound No. 48 of the invention):

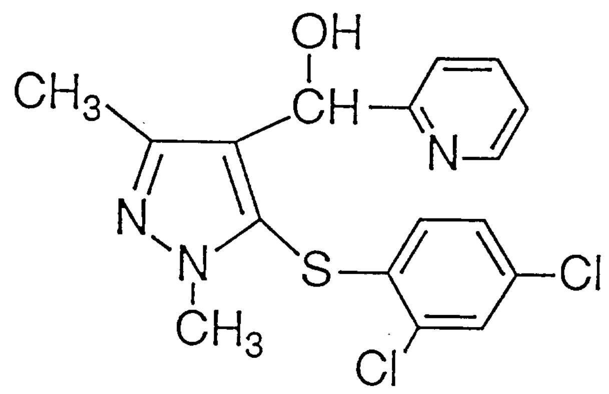

1.6 g (10.0 mmol) of 2-bromopyridine were dissolved in 80 ml of THF. This was cooled to -78°C, and 4.7 g (11.0 mmol) of n-butyl lithium/hexane solution (15 w/w %) were gradually and dropwise added thereto and stirred for one hour at the same temperature. Next, 20 ml of a THF solution containing 3.0 g (10.0 mmol) of 4-formyl-5-(2,4-dichlorophenylthio)-1,3-dimethylpyrazole were gradually and dropwise added thereto and then stirred for 15 hours at room temperature. The reaction solution was made to have pH of from 1 to 2 by adding a diluted hydrochloric acid thereto, and stirred for one hour. Next, this was adjusted to have pH of from 7 to 8, by adding an aqueous sodium hydrogencarbonate solution thereto, and was extracted three times each with 50 ml of ethyl acetate. The ethyl acetate layers were combined and dried with anhydrous sodium sulfate. The solvent was removed from this by distillation, and the residue was purified by silica gel column chromatography (developer: chloroform) to obtain 1.6 g of (5-(2,4-dichlorophenylthio)-1,3-dimethyl-4-pyrazolyl)-2-pyridylmethanol (Compound No. 48 of the invention) as semi-crystals. Its yield was 43 %.

Example 14:

Production of (5-(2,4-dichlorophenylthio)-1,3-dimethyl-4-pyrazolyl)-2-pyridylketone (Compound No. 49 of the invention):

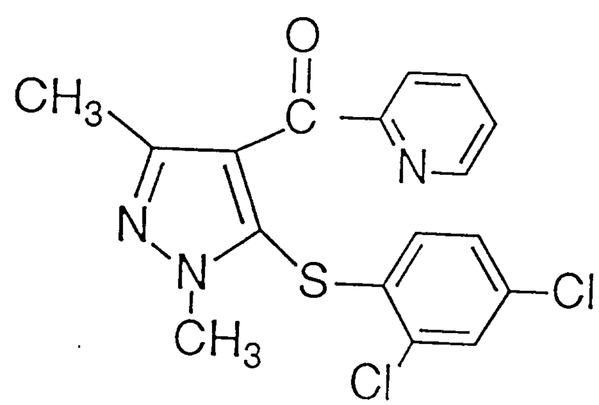

100 mg (0.26 mmol) of (5-(2,4-dichlorophenylthio)-1,3-dimethyl-4-pyrazolyl)-2-pyridylmethanol, produced in Example 13, were dissolved in 10 ml of dry methylene chloride. Next, 110 mg of active manganese dioxide were added thereto and stirred for 20 hours at room temperature. The manganese dioxide was removed from the reaction mixture by filtration through Celite, and the solvent was removed

from the resulting filtrate to obtain 95 mg of Compound No. 49 of the invention. This had a melting point of 136 to 137 °C.

Example 15:

Production of 5-(2,4-dichlorophenylthio)-1,3-dimethyl-4-(2-pyridylmethyl)pyrazole (Compound No. 50 of the invention):

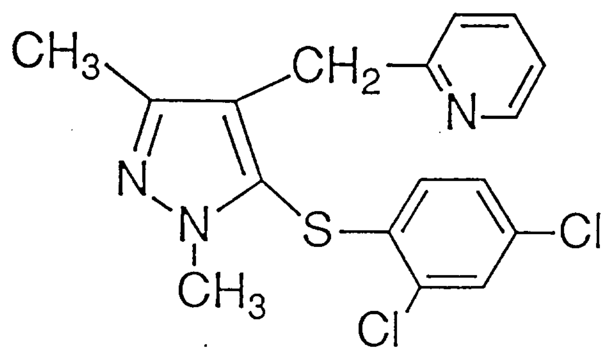

1.1 g (2.0 mmol) of diphosphorus tetraiodide were suspended in 30 ml of benzene and heated under reflux for 10 minutes. To this, dropwise added were 10 ml of a benzene solution containing 1.0 g of (5-(2,4-dichlorophenylthio)-1,3-dimethyl-4-pyrazolyl)-2-pyridylmethanol, produced in Example 13, and heated under reflux for 2 hours. After this was left to be cooled to room temperature, 5 ml of aqueous 10 % sodium hydrogensulfite were added thereto and stirred for one hour. This was extracted three times each with 30 ml of ethyl acetate, and the ethyl acetate layers were combined and dried on anhydrous sodium sulfate. The solvent was removed from this to obtain 0.3 g of 5-(2,4-dichlorophenylthio)-1,3-dimethyl-4-(2-pyridylmethyl)pyrazole (Compound No. 50 of the invention) as semi-crystals. Its yield was 31 %.

Example 16:

Production of methyl 3-chloro-5-(2,4-dichlorophenylthio)-1-methylpyrazole-4-carboxylate (Compound No. 140 of the invention):

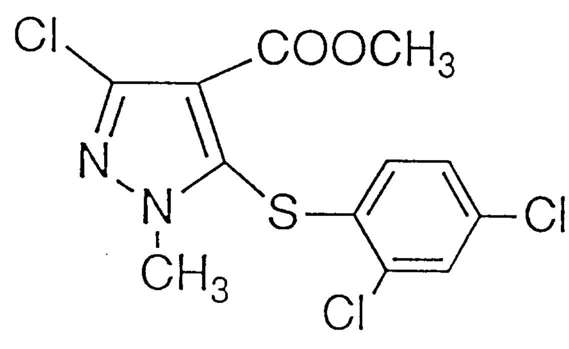

18 g of 2,4-dichlorobenzenethiol dissolved in 10 ml of DMF were added, little by little, to a solution of 100 ml of N,N-dimethylformamide containing 5.0 g of sodium hydride (55 %) suspended therein, while cooling with ice water, and then stirred at room temperature for 1.5 hours. To this solution, gradually and dropwise added was a solution comprising 21 g of methyl 3,5-dichloro-1-methylpyrazole-4-carboxylate and 20 ml of N,N-dimethylformamide, and stirred at room temperature for 5 days. The solvent was removed from the reaction mixture by distillation under reduced pressure, and 200 ml of water were added to the residue, which was then extracted three times each with a mixed solvent comprising 150 ml of diethyl ether

and 30 ml of ethyl acetate.

The organic layer was washed with 100 ml of water and then dried with anhydrous sodium sulfate. The solvent was removed from this by distillation under reduced pressure, and the crystals thus obtained were washed with normal hexane to obtain 22 g of methyl 3-chloro-5-(2,4-dichlorophenylthio)-1-methylpyrazole-4-carboxylate. Its yield was 62 %. The compound had a melting point of 99.0 to 101.0 °C.

Example 17:

Production of 3-chloro-5-(2,4-dichlorophenylthio)-1-methyl-4-(1-hydroxy-1-methylethyl)pyrazole:

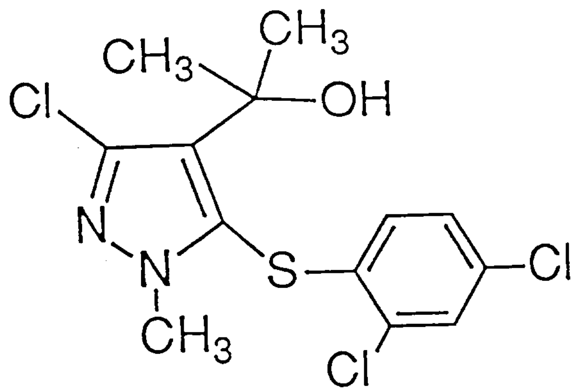

10 ml of diethyl ether were added to 1.8 g of magnesium, and a solution prepared by adding 10.7 g of methyl iodide to 20 ml of diethyl ether was gradually and dropwise added thereto, while stirring, to prepare a Grignard solution. To the solution, added were 30 ml of toluene and then a solution comprising 2.5 g of methyl 3-chloro-5-(2,4-dichlorophenylthio)-1-methylpyrazole-4-carboxylate and 25 ml of toluene, at room temperature. The reaction liquid was heated at 95 °C for 2.5 hours and then continuously stirred at room temperature for 15 hours. The reaction liquid was gradually poured into aqueous 1 N-hydrochloric acid solution, to which ice had been added, and a minimum amount of the aqueous hydrochloric acid solution enough to make the precipitates formed disappear was added thereto. Then, this was extracted three times each with 50 ml of diethyl ether. The organic layer was washed with water and dried with anhydrous sodium sulfate. Removing the solvent therefrom by distillation under reduced pressure, the intended product was obtained quantitatively. The product had a melting point of 50.5 to 51.0 °C.

Example 18:

Production of 3-chloro-5-(2,4-dichlorophenylsulfinyl)-4-hydroxy-1-methylpyrazole (Compound No. 144 of the invention):

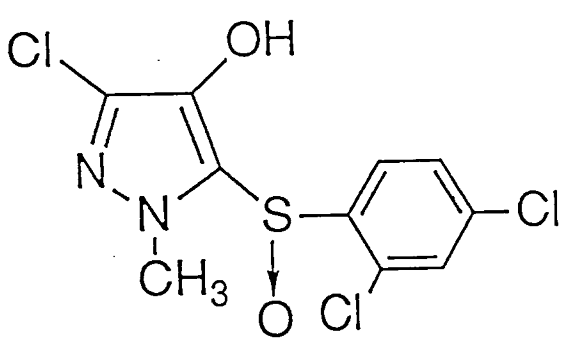

3.4 ml of aqueous 30 % hydrogen peroxide were kept at 15 ° C or lower by cooling them with ice water, and 4.4 ml of concentrated sulfuric acid were gradually added thereto. The previously-prepared mixture of aqueous hydrogen peroxide and concentrated sulfuric acid was gradually and dropwise added to a solution comprising 5 g of 3-chloro-5-(2,4-dichlorophenylthio)-1-methyl-4-(1-hydroxy-1-methylethyl)pyrazole and 14 ml of methylene chloride, while cooling the solution to 15 ° C with ice water. The resulting solution was stirred for 4 hours at room temperature, and 4.7 ml of aqueous 70 % sulfuric acid solution were added thereto and stirred for 15 hours at room temperature. The solution was poured into 100 ml of ice water, and aqueous 50 % sodium hydroxide solution was added thereto until the solution became alkaline. Then, 100 ml of chloroform were added thereto, resulting in liquid-liquid separation. Hydrochloric acid was added to the aqueous layer to make it acidic, and the acidic layer was extracted two times each with 100 ml of chloroform. The organic layer was dried with anhydrous sodium sulfate, then the solvent was removed therefrom by distillation under reduced pressure, and the resulting residue was crystallized from a mixed solution comprising normal hexane and benzene to obtain 3.4 g of the intended product. This had a melting point of 176.5 to 177.0 ° C.

Example 19:

Production of 3-chloro-5-(2,4-dichlorophenylthio)-4-hydroxy-1-methylpyrazole (Compound No. 116):

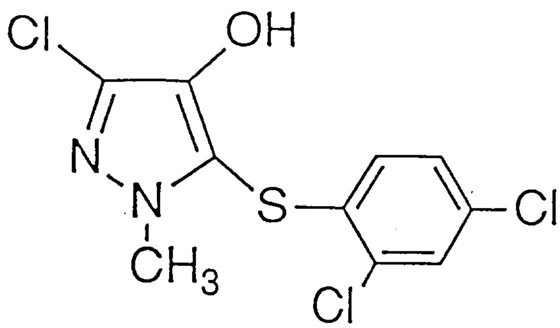

1.10 g of sodium iodide and 20 ml of acetonitrile were added to 0.30 g of 3-chloro-5-(2,4-dichlorophenylsulfinyl)-4-hydroxy-1-methylpyrazole and stirred. 0.40 g of phosphorus oxychloride were added to the resulting solution, while cooling it with ice water. This was restored to be at room temperature and the continuously stirred for one day. The solvent was removed from this solution by distillation under reduced pressure, and 50 ml of water were added thereto. This was then extracted three times each with 30 ml of chloroform. The chloroform layer was washed with 100 ml of aqueous 2 % sodium thiosulfate and then with water, and dried with anhydrous sodium sulfate.

The solvent was removed from this solution by distillation under reduced pressure to obtain 0.28 g of the intended compound as crystals. This had a melting point of 130.0 to 133.0 ° C.

Example 20:

Production of 3-chloro-5-(2,4-dichlorophenylthio)-1-methyl-4-(2-pyrimidyloxy)pyrazole (Compound No. 51 of the invention):

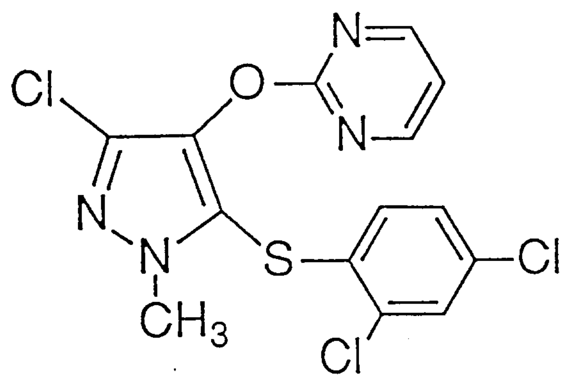

0.9 g of anhydrous potassium carbonate, 0.3 g of 2-chloropyrimidine and 30 ml of N,N-dimethylformamide were added to 0.8 g of 3-chloro-5-(2,4-dichlorophenylthio)-4-hydroxy-1-methylpyrazole and stirred at 70 °C for 10 hours. The solvent was removed from the resulting solution by distillation under reduced pressure, 100 ml of water were added thereto, and this was extracted three times each with 30 ml of chloroform. The chloroform layer was dried with anhydrous magnesium sulfate, and the solvent was removed therefrom by distillation under reduced pressure to obtain crystals. 1.0 g of the crystals was washed two times each with 20 ml of normal hexane to obtain 0.6 g of the intended compound. This had a melting point of 106.5 to 107.0 °C.

Compounds obtained according to the above-mentioned methods are shown in Table 2 and Table 3 below.

Table 2 (continued)

In compounds of:

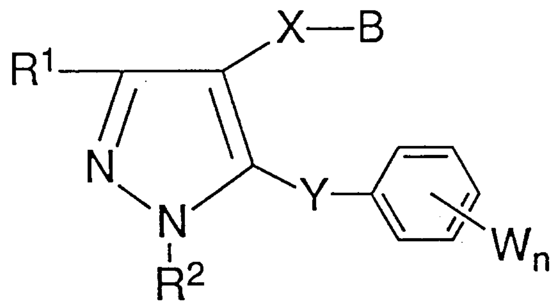

| No. | $R^1$ | $R^2$ | X | Y | B | $W_n$ | m.p. (℃) |
|---|---|---|---|---|---|---|---|
| 1 | $CH_3$ | $CH_3$ | NH | S | B1 | 4-$CH_3$ | 182.0-183.5 |
| 2 | $CH_3$ | $CH_3$ | NH | S | B1 | 4-Cl | 182.0-183.0 |
| 3 | $CH_3$ | $CH_3$ | NH | S | B1 | 4-Br | 125.0-126.0 |
| 4 | $CH_3$ | $CH_3$ | NH | S | B1 | 2-Cl | 172.0-174.0 |
| 5 | $CH_3$ | $CH_3$ | NH | S | B1 | 4-$CF_3$ | 155.0-157.0 |
| 6 | $CH_3$ | $CH_3$ | NH | S | B1 | 2,3-$Cl_2$ | 165.0-167.0 |
| 7 | $CH_3$ | $CH_3$ | NH | S | B1 | 2,4-$Cl_2$ | 155.0-157.0 |
| 8 | $CH_3$ | $CH_3$ | NH | S | B1 | 2,5-$Cl_2$ | 176.0-179.0 |
| 9 | $CH_3$ | $CH_3$ | NH | S | B1 | 3,4-$Cl_2$ | 92.0-94.0 |
| 10 | $CH_3$ | $CH_3$ | NH | S | B1 | 3,5-$Cl_2$ | 144.0-146.0 |
| 11 | $CH_3$ | $CH_3$ | NH | S | B1 | 2-F,4-Cl | 140.0-141.0 |

Table 2 (continued)

| No. | $R^1$ | $R^2$ | X | Y | B | $W_n$ | m.p. (℃) |
|---|---|---|---|---|---|---|---|
| 12 | $CH_3$ | $CH_3$ | NH | S | B1 | 2-F, 4-Br | 140.0-143.0 |
| 13 | $CH_3$ | $CH_3$ | NH | S | B1 | 3-Cl, 4-F | 115.0-116.0 |
| 14 | $CH_3$ | $CH_3$ | NH | S | B1 | 3-F, 4-$CH_3$ | 130.0-132.0 |
| 15 | $CH_3$ | $CH_3$ | NH | S | B1 | 2-Cl, 4-$CH_3$ | 135.5-137.0 |
| 16 | $CH_3$ | $CH_3$ | NH | S | B1 | 3-Cl, 4-$CH_3$ | 154.0-155.5 |
| 17 | $CH_3$ | $CH_3$ | NH | S | B1 | 3-$CH_3$, 4-Br | 100.0-102.0 |
| 18 | $CH_3$ | $CH_3$ | NH | S | B1 | 2, 3, 4-$Cl_3$ | 176.0-179.0 |
| 19 | $CH_3$ | $CH_3$ | N(CHO) | S | B1 | 4-Cl | oil |
| 20 | $CH_3$ | $CH_3$ | N(CHO) | S | B1 | 4-Br | 110.0-111.0 |
| 21 | $CH_3$ | $CH_3$ | N(CHO) | S | B1 | 2, 4-$Cl_2$ | vitreous substance |
| 22 | $CH_3$ | $CH_3$ | N(CHO) | S | B1 | 2, 5-$Cl_2$ | oil |
| 23 | $CH_3$ | $CH_3$ | N(CHO) | S | B1 | 3, 4-$Cl_2$ | oil |
| 24 | $CH_3$ | $CH_3$ | N(CHO) | S | B1 | 3-F, 4-$CH_3$ | 123.0-126.0 |
| 25 | $CH_3$ | $CH_3$ | N($CH_3$) | S | B1 | 2, 4-$Cl_2$ | 147.0-149.0 |

Table 2 (continued)

| No. | $R^1$ | $R^2$ | X | Y | B | $W_n$ | m.p. (℃) |
|---|---|---|---|---|---|---|---|
| 26 | $CH_3$ | $CH_3$ | $N(CH_2CH=CH_2)$ | S | B1 | 2,4-$Cl_2$ | 72.0-73.0 |
| 27 | $CH_3$ | $CH_3$ | $N(COCH_3)$ | S | B1 | 2,4-$Cl_2$ | 123.0-125.0 |
| 28 | $CH_3$ | $CH_3$ | $N(COOCH_3)$ | S | B1 | 2,4-$Cl_2$ | oil |
| 29 | $CH_3$ | $CH_3$ | NH | S | B2 | 2,4-$Cl_2$ | 135.0-136.0 |
| 30 | $CH_3$ | $CH_3$ | NH | S | B3 | 2,4-$Cl_2$ | 143.0-145.0 |
| 31 | $CH_3$ | $CH_3$ | NH | S | B13 | 2,4-$Cl_2$ | 191.0-192.0 |
| 32 | $CH_3$ | $CH_3$ | NH | S | B14 | 2,4-$Cl_2$ | 153.0-155.0 |
| 33 | $CH_3$ | $CH_3$ | NH | S | B15 | 2,4-$Cl_2$ | 199.0-200.0 |
| 34 | Cl | $CH_3$ | NH | S | B1 | 2,4-$Cl_2$ | 177.0-178.0 |
| 35 | Cl | $CH_3$ | NH | S | B1 | 3-F,4-$CH_3$ | 140.0-141.0 |
| 36 | Cl | $CH_3$ | NH | S | B1 | 2-Cl,4-$CH_3$ | 150.0-152.0 |
| 37 | Cl | $CH_3$ | NH | S | B1 | 3-Cl,4-$CH_3$ | 167.0-168.0 |
| 38 | Cl | $CH_3$ | NH | S | B1 | 3-Cl,4-$OCH_3$ | 170.0-171.0 |
| 39 | Cl | $CH_3$ | N(CHO) | S | B1 | 2,4-$Cl_2$ | 122.0-123.0 |
| 40 | Cl | $CH_3$ | N(CHO) | S | B1 | 3-F,4-$CH_3$ | 97.0-98.0 |
| 41 | Cl | $CH_3$ | N(CHO) | S | B1 | 2-Cl,4-$CH_3$ | 130.0-131.0 |
| 42 | Cl | $CH_3$ | N(CHO) | S | B1 | 3-Cl,4-$CH_3$ | 104.0-105.0 |
| 43 | Cl | $CH_3$ | N(CHO) | S | B1 | 3-Cl,4-$OCH_3$ | 117.0-118.0 |
| 44 | Cl | $CH_3$ | NH | O | B1 | 2,4-$Cl_2$ | 184.0-185.0 |
| 45 | $CH_3$ | $CH_3$ | S | NH | B1 | 4-Cl | 139.0-140.0 |

Table 2 (continued)

| No. | $R^1$ | $R^2$ | X | Y | B | $W_n$ | m.p. (℃) |
|---|---|---|---|---|---|---|---|
| 46 | $CH_3$ | $CH_3$ | S | NH | B1 | 2,4-$Cl_2$ | 110.0-112.0 |
| 47 | $CH_3$ | $CH_3$ | S | NH | B1 | 3-Cl,4-$CH_3$ | 142.0-142.0 |
| 48 | $CH_3$ | $CH_3$ | CH(OH) | S | B2 | 2,4-$Cl_2$ | semi-crystals |
| 49 | $CH_3$ | $CH_3$ | C=O | S | B2 | 2,4-$Cl_2$ | 136.0-137.0 |
| 50 | $CH_3$ | $CH_3$ | $CH_2$ | S | B2 | 2,4-$Cl_2$ | semi-crystals |
| 51 | Cl | $CH_3$ | O | S | B1 | 2,4-$Cl_2$ | 106.5-107.0 |
| 52 | Cl | $CH_3$ | O | S | B3 | 2,4-$Cl_2$ | 118.0-119.0 |
| 53 | Cl | $CH_3$ | O | S | B17 | 2,4-$Cl_2$ | 148.0-150.0 |
| 54 | Cl | $CH_3$ | O | S | B2 | 2,4-$Cl_2$ | 105.0-108.0 |
| 55 | Cl | $CH_3$ | O | S | B17 | 2-Cl | 128.0-129.5 |

In the above-mentioned table, B1 to B18 are represented by the following chemical structures:

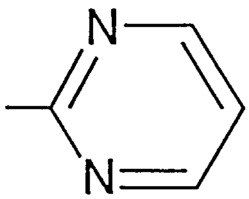
B1
,
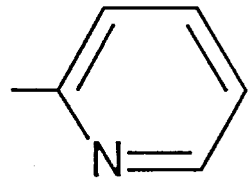
B2
,

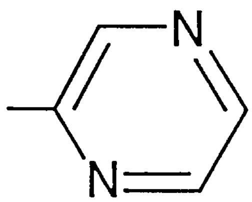
B3
,
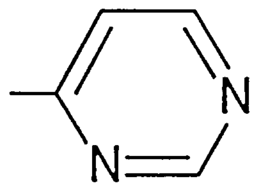
B4
,

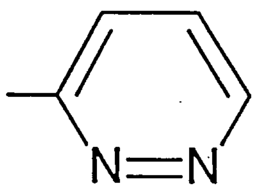
B5
,
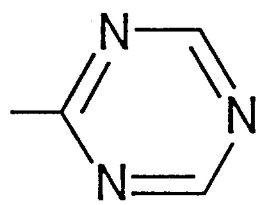
B6
,

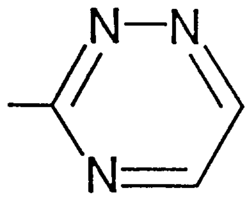

B7

,

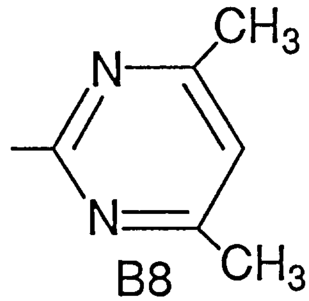

B8

,

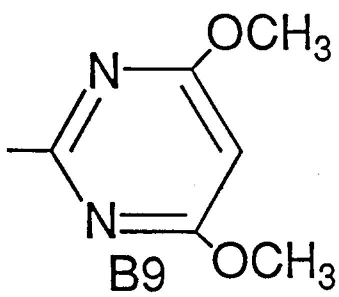

B9

,

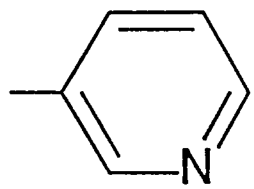

B10

,

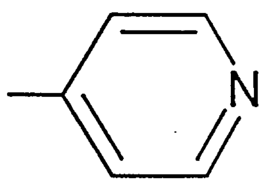

B11

,

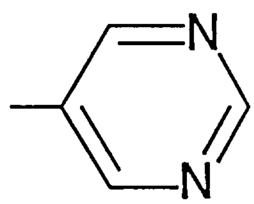

B12

,

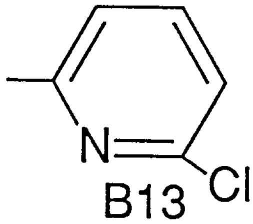
B13 ,
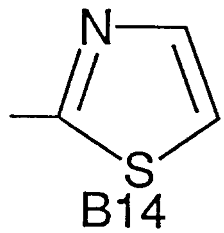
B14 ,

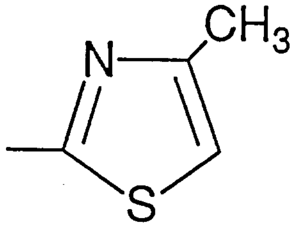
B15 ,
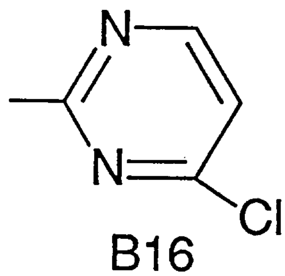
B16 ,

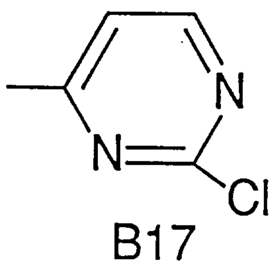
B17 ,
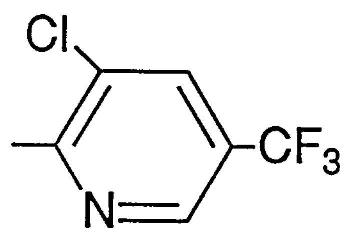
B18 ,

Table 3 (continued)

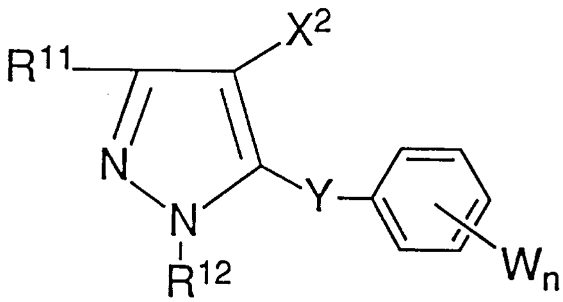

In compounds of:

| No. | $R^{11}$ | $R^{12}$ | Y | $W_n$ | $X^2$ | m.p. (℃) |
|---|---|---|---|---|---|---|
| 101 | $CH_3$ | $CH_3$ | S | 4-$CH_3$ | $NO_2$ | 75.0-77.0 |
| 102 | $CH_3$ | $CH_3$ | S | 4-Br | $NO_2$ | 125.0-126.0 |
| 103 | $CH_3$ | $CH_3$ | S | 4-$CF_3$ | $NO_2$ | 118.0-119.0 |
| 104 | $CH_3$ | $CH_3$ | S | 2-Cl-4-$CH_3$ | $NO_2$ | 72.0-75.0 |
| 105 | $CH_3$ | $CH_3$ | S | 3-Cl-4-$CH_3$ | $NO_2$ | 99.0-102.0 |
| 106 | $CH_3$ | $CH_3$ | S | 2-F-4-Br | $NO_2$ | 87.5-88.5 |
| 107 | $CH_3$ | $CH_3$ | S | 3-Cl-4-F | $NO_2$ | 81.0-82.0 |
| 108 | $CH_3$ | $CH_3$ | S | 2,3-$Cl_2$ | $NO_2$ | 134.0-135.0 |
| 109 | $CH_3$ | $CH_3$ | S | 2,3,4-$Cl_3$ | $NO_2$ | 100.0-105.0 |
| 110 | Cl | $CH_3$ | S | 2,4-$Cl_2$ | $NO_2$ | 106.0-108.0 |
| 111 | Cl | $CH_3$ | S | 2-Cl-4-$CH_3$ | $NO_2$ | 100.0-101.0 |

Table 3 (continued)

| No. | $R^{11}$ | $R^{12}$ | Y | $W_n$ | $X^2$ | m.p. (℃) |
|---|---|---|---|---|---|---|
| 112 | Cl | $CH_3$ | S | 3-Cl-4-$CH_3$ | $NO_2$ | 106.0-107.0 |
| 113 | Cl | $CH_3$ | S | 3-F-4-$CH_3$ | $NO_2$ | 78.0-79.0 |
| 114 | Cl | $CH_3$ | S | 3-Cl-4-$OCH_3$ | $NO_2$ | 105.0-107.0 |
| 115 | Cl | $CH_3$ | O | 2,4-$Cl_2$ | $NO_2$ | 122.0-123.0 |
| 116 | Cl | $CH_3$ | S | 2,4-$Cl_2$ | OH | 130.0-133.0 |
| 117 | $CH_3$ | $CH_3$ | S | 4-$CF_3$ | $NH_2$ | oil |
| 118 | $CH_3$ | $CH_3$ | S | 2-Cl-4-$CH_3$ | $NH_2$ | 93.0-95.0 |
| 119 | $CH_3$ | $CH_3$ | S | 3-Cl-4-$CH_3$ | $NH_2$ | oil |
| 120 | $CH_3$ | $CH_3$ | S | 2-F-4-Br | $NH_2$ | oil |
| 121 | $CH_3$ | $CH_3$ | S | 2,3-$Cl_2$ | $NH_2$ | 72.0-73.0 |
| 122 | $CH_3$ | $CH_3$ | S | 2,3,4-$Cl_3$ | $NH_2$ | 91.0-96.0 |
| 123 | Cl | $CH_3$ | S | 2,4-$Cl_2$ | $NH_2$ | 86.0-87.0 |
| 124 | Cl | $CH_3$ | S | 2-Cl-4-$CH_3$ | $NH_2$ | 72.0-73.0 |
| 125 | Cl | $CH_3$ | S | 3-Cl-4-$CH_3$ | $NH_2$ | oil |
| 126 | Cl | $CH_3$ | S | 3-F-4-$CH_3$ | $NH_2$ | oil |
| 127 | Cl | $CH_3$ | S | 3-Cl-4-$OCH_3$ | $NH_2$ | 111.0-112.0 |
| 128 | Cl | $CH_3$ | O | 2,4-$Cl_2$ | $NH_2$ | 87.0-88.0 |
| 129 | $CH_3$ | $CH_3$ | S | 4-$CF_3$ | NHCHO | 145.0-146.0 |
| 130 | $CH_3$ | $CH_3$ | S | 2,3-$Cl_2$ | NHCHO | 128.0-132.0 |
| 131 | $CH_3$ | $CH_3$ | S | 2,3,4-$Cl_3$ | NHCHO | 150.0-156.0 |

Table 3 (continued)

| No. | $R^{11}$ | $R^{12}$ | Y | $W_n$ | $X^2$ | m.p. (℃) |
|---|---|---|---|---|---|---|
| 132 | Cl | $CH_3$ | S | 2,4-$Cl_2$ | NHCHO | 166.0-167.0 |
| 133 | Cl | $CH_3$ | S | 2-Cl-4-$CH_3$ | NHCHO | 115.0-117.0 |
| 134 | Cl | $CH_3$ | S | 3-Cl-4-$CH_3$ | NHCHO | 121.0-123.0 |
| 135 | Cl | $CH_3$ | S | 3-F-4-$CH_3$ | NHCHO | 109.0-111.0 |
| 136 | Cl | $CH_3$ | S | 3-Cl-4-$OCH_3$ | NHCHO | 166.0-167.0 |
| 137 | Cl | $CH_3$ | O | 2,4-$Cl_2$ | NHCHO | 164.0-165.0 |
| 138 | $CH_3$ | $CH_3$ | S | 2,4-$Cl_2$ | CHO | 64.0-65.0 |
| 139 | Cl | $CH_3$ | S | 4-$CH_3$ | $COOCH_3$ | 77.0-78.0 |
| 140 | Cl | $CH_3$ | S | 2,4-$Cl_2$ | $COOCH_3$ | 99.0-101.0 |
| 141 | $CH_3$ | $CH_3$ | S | 2,4-$Cl_2$ | $NHCSNH_2$ | 221.0-222.0 |
| 142 | $CH_3$ | $CH_3$ | S | 2,4-$Cl_2$ | $NH_2$ | oil |
| 143 | $CH_3$ | $CH_3$ | S | 2,4-$Cl_2$ | NHCHO | 124.0-127.0 |
| 144 | Cl | $CH_3$ | SO | 2,4-$Cl_2$ | OH | 176.5-177.0 |
| 145 | Cl | $CH_3$ | S | 2-Cl | $COOCH_3$ | 72.0-75.0 |
| 146 | Cl | $CH_3$ | SO | 2-Cl | OH | 125.0-131.0 |

When the compounds of the present invention are used as phytopathogenic microbicides, in general, they may be mixed with suitable carriers, for example, solid carriers such as clay, talc, bentonite, diatomaceous earth, etc., or liquid carriers such as water, alcohols (e.g., methanol, ethanol, etc.), aromatic hydrocarbons (e.g., benzene, toluene, xylene, etc.), chlorinated hydrocarbons, ethers, ketones, esters (e.g., ethyl acetate, etc.), acid amides (e.g., dimethylformamide, etc.), and, if desired, additives such as emulsifying agent, dispersing agent, suspending agent, penetrating agent, vehicle, stabilizer, etc. may be added thereto, to formulate any desired practical preparations such as liquid, emulsion, wettable powder, power, granules, flowable preparation, etc.

If desired, other herbicides, various insecticides, microbicides, plant growth regulators, agonists, etc. may be added to the compounds of the present invention when they are formulated into preparations or when the formulated preparations are sprayed.

The dose of the compounds of the present invention varies, depending on the place to which they are applied, the time when they are applied, the method how to apply them, the plant diseases which are

desired to be overcome by them, the crops which are desired to be protected from phytopathogenic microbes by them, etc. In general, the effective dose is approximately from 0.005 to 50 kg per hectare.

Next, examples of formulations of microbicides containing the compounds of the present invention as active ingredients are shown below, which, however, are not limitative. In the following formulation examples, "parts" are by weight.

Formulation Example 1: Emulsion

| | |
|---|---|
| Compound of the invention | 20 parts |
| Xylene | 55 parts |
| Cyclohexanone | 20 parts |
| Solpol 2680 (mixture of nonionic surfactant and anionic surfactant; trade name by Toho Chemical Industry Co.) | 5 parts |

The above ingredients were uniformly mixed to an emulsion. The emulsion is diluted to 1/50 to 1/20000 before use, and the dilution is sprayed in an amount of from 0.005 to 50 kg/hectare as the active ingredient.

Formulation Example 2: Wettable Powder

| | |
|---|---|
| Compound of the invention | 25 parts |
| Zeaklite PFP (mixture of kaolinite and sericite; trade name by Zeaklite Industry Co.) | 66 parts |
| Solpol 5039 (anionic surfactant; trade name by Toho Chemical Industry Co.) | 4 parts |
| Carplex #80 (white carbon; trade name by Shionogi & Co.) | 3 parts |
| Calcium Lignin Sulfonate | 2 parts |

The above ingredients were uniformly mixed and powdered to a wettable powder. The wettable powder is diluted to 1/50 to 1/20000 before use, and the dilution is sprayed in an amount of from 0.005 to 50 kg/hectare as the active ingredient.

Formulation Example 3: Powder

| | |
|---|---|
| Compound of the invention | 3.0 parts |
| Carplex #80 (white carbon; trade name by Shionogi & Co.) | 0.5 parts |
| Clay | 95 parts |
| Diisopropyl Phosphate | 1.5 parts |

The above ingredients were uniformly mixed and powdered to a powder. The powder is sprayed in an amount of from 0.005 to 50 kg/hectare as the active ingredient.

Formulation Example 4: Granules

| | |
|---|---|
| Compound of the invention | 5 parts |
| Bentonite | 54 parts |
| Talc | 40 parts |
| Calcium Lignin Sulfonate | 1 part |

The above ingredients were uniformly mixed and powdered. A small amount of water was added thereto, stirred, granulated through an extrusion granulator and dried to form granules. The granules are sprinkled in an amount of from 0.005 to 50 kg/hectare as the active ingredient.

Formulation Example 5: Flowable Liquid

| | |
|---|---|
| Compound of the invention | 25 parts |
| Solpol 3353 (nonionic surfactant; trade name by Toho Chemical Industry Co.) | 10 parts |
| Runox 1000C (anionic surfactant; trade name by Toho Chemical Industry Co.) | 0.5 parts |
| Aqueous Solution of 1 % xanthan gum (natural polymer) | 20 parts |
| Water | 44.5 parts |

The above ingredients except the compound of the invention were uniformly dissolved, and the compound of the invention was added thereto and well stirred. The resulting mixture was then wet-milled in a sand mill to obtain a flowable liquid. The flowable liquid is diluted to 1/50 to 1/20000 before use, and the dilution is sprayed in an amount of from 0.005 to 50 kg/hectare as the active ingredient.

Phytopathogens to cause plant diseases, which shall be exterminated by the compounds of the present invention, include Pyricularia oryzae, Cochliobolus miyabeanus, Rhizoctonia solani, Erysiphe graminis f. sp. hordei, f. sp. tritici, Pyrenophora graminea, Phyrenophora teres, Gibberella zeae, Puccinia striiformis, P. graminis, P. recondita, P. hordei, Typhula sp., Micronectriella nivais, Ustilago tritici, U. nuda, Pseudocercosporella herpotrichoides, Rhynchosporium secalis, Septoria tritici, Leptosphaeria nodorum, Diaporthe citri, Blsinoe fawcetti, Penicillium digitatum, P. italicum, Sclerotinia mali, Valsa mali, Podosphaera leucotricha, Alternaria mali, Venturia inaequalis, Venturia nashicola, Alternaria kikuchiana, Gymnosporangium haraeanum, Sclerotinia cinerea, Cladosporium carpophilum, Rhomopsis sp., Plasmopara viticola, Elsinoe ampelina, Glomerella cingulata, Uncinula necator, Phakopsora ampelopsidis, Gloeosporium kaki, Cercospora kaki, Mycosphaerella nawae, Pseudoperenospora cubensis, Colletotrichum lagenarium, Sphaerotheca fuliginea, Mycosphaerella melonis, Phytophthora infestans, Alternaria solani, Cladosporium fulvam, Phomopsis vexans, Erysiphe cichoracoarum, Alternaria japonica, Cerocosporella brassicae, Puccinia allii, Cercospora kikuchii, Elsinoe glycines, Diaporthe phaseololum, Colletotrichum lindemuthianum, Mycosphaerella personatum, Cercospora arachidicola, Erysiphe pisi, Alternaria solani, Sphaerotheca humuli, Exobasidium reticulatum, Elsinoe leucospila, Alternaria longipes, Erysiphe cichoracearum, Colletotrichum tabacum, Cercospora beticola, Diplocarpon rosae, Sphaerotheca pannosa, Septoria chrysanthemiindici, Puccinia horiana, Botrytis cinerea, Sclerotinia sclerotiorum, etc. The usefulness of the compounds of the present invention will be verified by means of the following test examples, which, however, are not intended to restrict the scope of the present invention.

Test Example 1: Test for Anti-microbial Effect against Botrytis cinerea

A 500-ppm liquid preparation prepared by diluting an emulsion containing one compound of the present invention was sprayed over two to three-leaves tomato seedlings (variety: Fukuju) that had grown in 7 cm-diameter pots, using a spray gum, in an amount of 20 ml/pot.

On the day following the spraying, a suspension of spores of Botrytis cinerea (containing 1.0 % glucose and 2.5 % yeast extract; × 150•40 spores/visual field) was sprayed over the plants, and the plants were then placed in an incubator at a temperature of 25°C and a humidity of 95 % or more for 5 days, whereupon the proportion of the spots to the infected leaves was measured. Based on the following equation, the anti-microbial value of the test compound was calculated.

Anti-microbial Value = [1-(areal proportion of spots in treated group/areal proportion of spots in non-treated control group)] × 100

The following compounds of the present invention had an anti-microbial value of 100 in the above-mentioned test.
No. 4, No. 5, No. 7, No. 8, No. 9, No. 10, No. 11, No. 12, No. 14, No. 15, No. 16, No. 17, No. 18, No. 30, No. 32, No. 34, No. 35, No. 36, No. 37, No. 39, No. 40, No. 45, No. 46, No. 48, No. 50, No. 51, No. 52, No. 53, No. 54, No. 55.

Test Example 2: Test for Anti-microbial Effect Against Rhizoctonia solani

A 500-ppm liquid preparation prepared by diluting an emulsion containing one compound of the present invention was applied to the vicinities of the roots of three to four-leaves rice seedlings (variety: Nipponbare) that had brown in 5 cm-diameter pots, in an amount of 5 ml/pot. Immediately after the application, the same dilution was sprayed over the seedlings in an amount of 15 ml/pot. On three days following the treatment, rice hulls infected with Rhizoctonia solani were put in the vicinities of the roots of the seedlings so that the seedlings were infected with the phytopathogenic cells.

After the infection, the pots were placed in an incubator at a temperature of 28°C and a humidity of 95 % or more. On five days following the infection, the height of the spotted area in each plant from the surface of the earth was measured. Based on the following equation, the anti-microbial value of the test compound was calculated.

Anti-microbial Value = [1-(height of spotted area in treated group/height of spotted area in non-treated control group)] × 100

The following compounds of the present invention had an anti-microbial value of 100 in the above-mentioned test.

No. 1, No. 2, No. 4, No. 5, No. 6, No. 7, No. 8, No. 9, No. 10, No. 11, No. 12, No. 13, No. 14, No. 15, No. 16, No. 18, No. 19, No. 20, No. 21, No. 22, No. 23, No. 24, No. 25, No. 26, No. 27, No. 28, No. 29, No. 34, No. 35, No. 36, No. 37, No. 38, No. 39, No. 40, No. 41, No. 43, No. 45, No. 46, No. 48, No. 49, No. 50, No. 51, No. 52, No. 53, No. 54, No. 55.

INDUSTRIAL APPLICATION

The compounds of the present invention are novel and have excellent phytopathogenic microbicidal activity, while causing no damage to useful crops. Accordingly, these are useful as phytopathogenic microbicides.

## Claims

**1.** Substituted pyrazole derivatives of a general formula [1], and their salts:

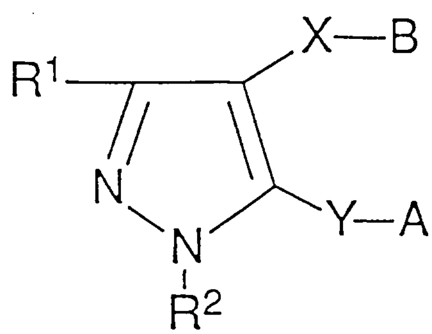

[1]

wherein $R^1$ represents a hydrogen atom, a halogen atom, an alkyl group, an alkoxy group, an alkylthio group, a haloalkyl group, a cyano group, an alkoxycarbonyl group, or an unsubstituted or substituted phenyl group;

$R^2$ represents a hydrogen atom, an alkyl group, a haloalkyl group, an unsubstituted or substituted phenyl group, an unsubstituted or substituted phenylalkyl group, $-COR^6$, or $-SO_2R^7$;

X represents an oxygen atom, -S-, -SO-, $-SO_2-$, $-N(R^3)-$, -CO-, or $-C(R^4)(R^5)-$;

Y represents an oxygen atom, -S-, -SO-, $-SO_2-$, or $-N(R^3)-$;

$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, an unsubstituted or substituted phenylalkyl group, $-COR^6$, or $-SO_2R^7$;

$R^4$ and $R^5$ each independently represent a hydrogen atom, a halogen atom, an alkyl group, a haloalkyl group, an alkenyl group, an alkynyl group, or $-OR^8$;

$R^8$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, an unsubstituted or substituted phenylalkyl group, $-COR^6$, or $-SO_2R^7$;

$R^6$ represents a hydrogen atom, an alkyl group, a haloalkyl group, an unsubstituted or substituted

phenyl group, an unsubstituted or substituted phenylalkyl group, an alkoxy group, or -N($R^9$)($R^{10}$);
$R^7$ represents an alkyl group, a haloalkyl group, an unsubstituted or substituted phenyl group, or -N-($R^9$)($R^{10}$);
$R^9$ and $R^{10}$ each independently represent a hydrogen atom, an alkyl group, or an unsubstituted or substituted phenyl group;
A represents an unsubstituted or substituted phenyl group; and
B represents an unsubstituted or substituted heterocyclic group.

2. Pyrazole derivatives and their salts as claimed in claim 1, in which X is an oxygen atom or - N($R^3$)-.

3. Pyrazole derivatives and their salts as claimed in claim 2, in which Y is -S- or an oxygen atom.

4. Pyrazole derivatives and their salts as claimed in claim 3, in which $R^1$ is a lower alkyl group or a halogen atom, $R^2$ is a lower alkyl group, X is an oxygen atom or -N($R^3$)-, Y is -S-, and B is an unsubstituted or substituted pyridyl group or an unsubstituted or substituted pyrimidyl group.

5. A method for producing substituted pyrazole derivatives of a general formula [4]:

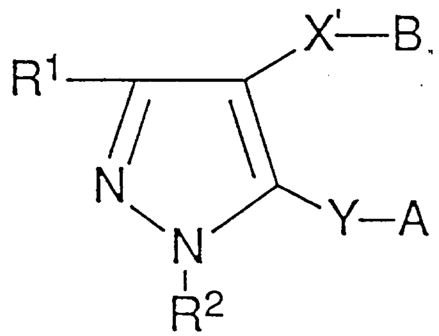

[4]

wherein $R^1$ represents a hydrogen atom, a halogen atom, an alkyl group, an alkoxy group, an alkylthio group, a haloalkyl group, a cyano group, an alkoxycarbonyl group, or an unsubstituted or substituted phenyl group;
$R^2$ represents a hydrogen atom, an alkyl group, a haloalkyl group, an unsubstituted or substituted phenyl group, an unsubstituted or substituted phenylalkyl group, -$COR^6$, or -$SO_2R^7$;
X' represents an oxygen atom, -S-, or -N($R^3$)-;
Y represents an oxygen atom, -S-, -SO-, -$SO_2$-, or -N($R^3$)-;
$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, an unsubstituted or substituted phenylalkyl group, -$COR^6$, or -$SO_2R^7$;
$R^6$ represents a hydrogen atom, an alkyl group, a haloalkyl group, an unsubstituted or substituted phenyl group, an unsubstituted or substituted phenylalkyl group, or an alkoxy group;
$R^7$ represents an alkyl group, a haloalkyl group, or an unsubstituted or substituted phenyl group;
A represents an unsubstituted or substituted phenyl group; and
B represents an unsubstituted or substituted heterocyclic group,
by reacting a substituted pyrazole of a general formula [2]:

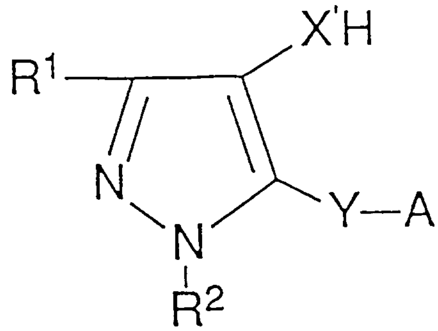

[2]

wherein $R^1$ represents a hydrogen atom, a halogen atom, an alkyl group, an alkoxy group, an alkylthio group, a haloalkyl group, a cyano group, an alkoxycarbonyl group, or an unsubstituted or substituted phenyl group;
$R^2$ represents a hydrogen atom, an alkyl group, a haloalkyl group, an unsubstituted or substituted phenyl group, an unsubstituted or substituted phenylalkyl group, $-COR^6$, or $-SO_2R^7$;
X' represents an oxygen atom, -S-, or $-N(R^3)-$;
Y represents an oxygen atom, -S-, -SO-, $-SO_2-$, or $-N(R^3)-$;
$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, an unsubstituted or substituted phenylalkyl group, $-COR^6$, or $-SO_2R^7$;
$R^6$ represents a hydrogen atom, an alkyl group, a haloalkyl group, an unsubstituted or substituted phenyl group, an unsubstituted or substituted phenylalkyl group, or an alkoxy group;
$R^7$ represents an alkyl group, a haloalkyl group, or an unsubstituted or substituted phenyl group;
A represents an unsubstituted or substituted phenyl group, and a heterocyclic compound of a general formula [3]:

L-B [3]

wherein L represents a split-off group; and
B represents an unsubstituted or substituted heterocyclic group.

6. A phytopathogenic microbicide containing one or more chosen from among substituted pyrazole derivatives and their salts as claimed in claim 1, as active ingredients.

7. Substituted pyrazole derivatives of a general formula [5], and their salts:

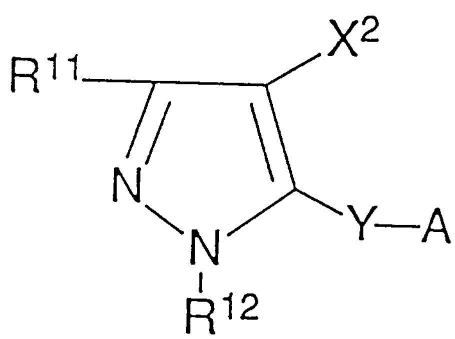

[5]

wherein $R^{11}$ represents a hydrogen atom, a halogen atom, an alkyl group, an alkoxy group, an alkylthio group, a haloalkyl group, a cyano group, an alkoxycarbonyl group, or an unsubstituted or substituted phenyl group;
$R^{12}$ represents a hydrogen atom, an alkyl group, a haloalkyl group, an unsubsituted or substituted phenyl group, an unsubstituted or substituted phenylalkyl group, $-COR^6$, or $-SO_2R^7$;
Y represents an oxygen atom, -S-, -SO-, $SO_2-$, or $-N(R^3)-$;

$X^2$ represents a nitro group, $-NH(R^3)$, -OH, -SH, -CHO, an alkoxycarbonyl group, or $-NHC(=S)NH_2$;
$R^3$ represents a hydrogen atom, an alkyl group, a haloalkyl group, an alkenyl group, an alkynyl group, an alkoxyalkyl group, a cyanoalkyl group, an alkylcarbonylalkyl group, an alkoxycarbonylalkyl group, a nitroso group, an amino group, a substituted or unsubstituted phenylalkyl group, $-COR^6$, or $-SO_2R^7$;
$R^6$ represents a hydrogen atom, an alkyl group, a haloalkyl group, a substituted or unsubstituted phenyl group, a substituted or unsubstituted phenylalkyl group, an alkoxy group, or $-N(R^9)(R^{10})$;
$R^7$ represents an alkyl group, a haloalkyl group, an unsubstituted or substituted phenyl group, or $-N(R^9)(R^{10})$;
$R^9$ and $R^{10}$ each independently represent a hydrogen atom, an alkyl group, or an unsubstituted or substituted phenyl group;
A represents an unsubstituted or substituted phenyl group.

**8.** Substituted pyrazole derivatives and their salts as claimed in claim 7, in which Y is -S- or an oxygen atom.

**9.** Substituted pyrazole derivatives and their salts as claimed in claim 8, in which $R^{11}$ is a halogen atom or an alkyl group, $R^{12}$ is an alkyl group, and $X^2$ is a nitro group, $-NH(R^3)$ or -OH.

## INTERNATIONAL SEARCH REPORT

International application No.
PCT/JP93/00065

**A. CLASSIFICATION OF SUBJECT MATTER** Int. $Cl^5$ C07D231/18, 231/20, 231/44, 231/46, 401/06, 401/10, 401/12, 403/06, 403/10, 403/12, 413/06, 413/10, 413/12, 417/12, A01N43/40, 43/54, 43/58, 43/60, 43/66, 43/707

According to International Patent Classification (IPC) or to both national classification and IPC

**B. FIELDS SEARCHED**

Minimum documentation searched (classification system followed by classification symbols) Int. $Cl^5$ C07D231/18, 231/20,231/44,231/46,401/06,401/10,401/12,403/06,403/10,403/12 413/06,413/10,413/12,417/12,A01N43/40,43/54,43/58,43/60,43/66,43/707

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 1-201630 (Ricoh Co., Ltd.), August 14, 1989 (14. 08. 89), (Family: none) | 1-9 |
| A | JP, A, 3-163189 (Ricoh Co., Ltd.), July 15, 1991 (15. 07. 91), (Family: none) | 1-9 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| April 5, 1993 (05. 04. 93) | April 20, 1993 (20. 04. 93) |
| Name and mailing address of the ISA/ Japanese Patent Office | Authorized officer |
| Facsimile No. | Telephone No. |